# EUROPÄISCHE PATENTSCHRIFT

(11) **EP 0 359 864 B1**
(45) Veröffentlichungstag und Bekanntmachung des Hinweises auf die Patenterteilung: **01.12.1993**
(21) Anmeldenummer: 88115716.8
(22) Anmeldetag: 23.09.1988
(51) Int. Cl.: G01R 33/00, G01R 33/035, A61B 5/00

(54) **Einrichtung und Verfahren zur Messung von schwachen, orts- und zeitabhängigen Magnetfeldern**
Apparatus and method for the measurement of weak magnetic fields dependent upon position and time
Dispositif et procédé de mesure de champs magnétiques faibles, dépendant de la position et du temps

(43) Veröffentlichungstag der Anmeldung: 28.03.1990
(73) Patentinhaber: SIEMENS AKTIENGESELLSCHAFT, 80333 München (DE)
(72) Erfinder: Hoenig, Hans Eckhardt, Dr.habil., D-8520 Erlangen (DE); Reichenberger, Helmut, Dipl.-Phys., Dr.rer.nat., D-8521 Eckental (DE); Schneider, Siegfried, Dipl.-Phys., Dr.rer.nat., D-8520 Erlangen (DE)

(56) Entgegenhaltungen:
- EP-A- 0 277 283
- DE-U- 8 715 844
- IEEE TRANSACTIONS ON MAGNETICS, Band MAG-18, Nr. 1, Januar 1982, Seiten 260-270, IEEE, New York, US; V.O. KELHÄ et al.: "Design, construction, and performance of a large-volume magnetic shield"
- REVIEW OF SCIENTIFIC INSTRUMENTS, Band 58, Nr. 11, November 1987, Seiten 2145-2156, American Instititute of Physics, New York, US; J. KNUUTILA et al.: "Large-area low-noise seven-channel dc SQUID magnetometer for brain research"
- BIOMAGNETISM, PROCEEDINGS THIRD INTERNATIONAL WORKSHOP ON BIOMAGNETISM, Berlin, Mai 1980, Seiten 51-79, Walter de Gruyter; A. MAGER: "The Berlin magnetically shielded room (BMSR), Section A: design and construction"

## Beschreibung

Die Erfindung betrifft eine Einrichtung zur Messung von schwachen, orts- und zeitabhängigen Magnetfeldern, die von einer im Inneren eines Untersuchungsobjektes befindlichen Quelle ausgehen, welche Einrichtung aufweist
a) eine Lagerungseinrichtung zur Aufnahme des Untersuchungsobjektes,
b) eine Sensoranordnung aufweisend ein Array mit wenigstens zehn Gradiometern erster Ordnung, wobei jedes Gradiometer eine Feldspule und eine entsprechende Kompensationsspule aufweist und die Feldspulen der Gradiometer in einer Sensorfläche angeordnet sind, ein Array von einer der Anzahl der Gradiometer entsprechenden Anzahl von DC-SQUIDs, wobei jedes Gradiometer mit einem der DC-SQUIDs induktiv gekoppelt ist, und ein die Gradiometer und die entsprechenden DC-SQUIDs enthaltendes Gefäß, in dem eine Temperatur herrscht, bei der die DC-SQUIDs und die Gradiometer supraleitend sind,
c) eine Halterung für die Sensoranordnung,
d) Mittel zur Verstellung der Lagerungseinrichtung und der Sensoranordnung relativ zueinander derart, daß die Sensoranordnung auf gewünschte Zonen des Untersuchungsobjektes ausrichtbar ist,
e) eine die Lagerungseinrichtung und die Sensoranordnung umschließende Kammer zur Abschirmung magnetischer Felder, wobei
f) die magnetische Abschirmkammer für magnetische Wechselfelder mit einer Frequenz von 0,5 Hz einen Abschirmfaktor von wenigstens 10, für magnetische Wechselfelder mit einer Frequenz von 5 Hz einen Abschirmfaktor von wenigstens 100 und für magnetische Wechselfelder mit einer Frequenz von 50 Hz und darüber einen Abschirmfaktor von wenigstens 1 000 aufweist, und
g) eine elektronische Einrichtung zur Verstärkung und Auswertung der Signale der Gradiometer, welche eine mit den DC-SQUIDs verbundene Verstärkeranordnung, einen mit der Verstärkeranordnung verbundenen Analog/Digital-Wandler und eine mit diesem verbundene elektronische Datenverarbeitungsanlage aufweist, wobei die Verstärkeranordnung eine der Anzahl der Gradiometer entsprechende Anzahl von Verstärkerkanälen besitzt, von denen jeder mit einem der DC-SQUIDs verbunden ist, und die Datenverarbeitungsanlage eine Ausgabeeinrichtung für Ergebnisse der Auswertung der Signale der Gradiometer aufweist.

Die Erfindung betrifft ebenfalls ein Betriebsverfahren einer derartigen Einrichtung.

Einrichtungen zur Messung von schwachen, orts- und zeitabhängigen Magnetfeldern gewinnen insbesondere für die Messung biomagnetischer Signale, d.h. von Signalen, die von im Körper eines Lebewesens befindlichen Quellen ausgehen, für die medizinische Diagnostik immer größere Bedeutung (vgl. Zeitschrift "Bild der Wissenschaft", Heft 8, 1986, Seiten 76 bis 83). Derartige Einrichtungen müssen in der Lage sein, die äußerst schwachen biomagnetischen Signale, z.B. die vom menschlichen Gehirn oder vom menschlichen Herzen ausgehenden Magnetfelder, deren Feldstärke in der Größenordnung von 10⁻¹² T und darunter liegt, meßtechnisch zu erfassen. Diese Signale werden in der medizinischen Diagnostik zur Anfertigung von Magnetoencephalogrammen (MEG) und Magnetokardiogrammen (MKG) benötigt, die gegenüber Elektroencephalogrammen (EEG) und Elektrokardiogrammen (EKG) den Vorteil bieten, daß die hier auftretenden Verzerrungen, die durch den Durchgang der zu erfassenden Ströme durch das menschliche Gewebe verursacht werden, deutlich geringer sind. Von besonderem Interesse für die experimentielle Forschung und die Diagnostik sind auch die z.B. durch akustische und optische Stimulierung der Sinne eines als Untersuchungsobjekt vorgesehenen Lebewesens in dessen Gehirn hervorgerufenen magnetischen Felder.

Bekannte biomagnetische Meßeinrichtungen besitzen gewöhnlich eine Lagerungseinrichtung zur Aufnahme des Untersuchungsobjektes, eine Sensoranordnung zur Messung von Magnetfeldern, eine Halterung für die Sensoranordnung, Mittel zur Verstellung der Lagerungseinrichtung und der Sensoranordnung relativ zueinander, und eine elektronische Einrichtung zur Verstärkung und Auswertung der von der Sensoranordnung stammenden Signale, welche eine Datenverarbeitungsanlage zur Auswertung der gewonnenen Signale mit einer Ausgabeeinrichtung für Meßergebnisse umfaßt. Dabei kann eine die Lagerungseinrichtung und die Sensoranordnung umschließende Kammer zur Abschirmung magnetischer Felder (Abschirmkammer) vorgesehen sein. In der Regel weist die Sensoranordnung ein oder mehrere Gradiometer (Feldmeßspulen mit zugeordneten Kompensationsspulen) erster oder höherer Ordnung, eine der Anzahl der Gradiometer entsprechende Anzahl von SQUIDs (Superconducting Quantum Interference Device), wobei jedes Gradiometer mit einem der SQUIDs induktiv gekoppelt ist, und ein die Gradiometer und die SQUIDs enthaltendes Gefäß, einen sogenannten Kryostaten auf, in dem eine Temperatur herrscht, bei der die SQUIDs und die Gradiometer supraleitend sind. In der Regel ist der Kryostat mit flüssigem Helium gefüllt, d.h. in seinem Inneren liegt eine Temperatur von 4,2 K vor.

Mit Einrichtungen dieser Art ist es grundsätzlich möglich, die örtliche Feldstärke des von einer Quelle ausgehenden Magnetfeldes in Abhängigkeit von der Zeit oder durch geeignete Auswertung der von der Sensoranordnung stammenden Signale unter Berücksichtigung der Lage der Sensoranordnung relativ zu dem Untersuchungsobjekt z.B. die räumliche Lage einer im Inneren des Untersuchungsobjektes befindlichen Quelle eines Magnetfeldes zu bestimmen, und zwar mittels geeigneter Rechenverfahren, die auf der Datenverarbeitungsanlage, der die Signale der Sensoranordnung zugeführt sind, ablaufen.

Dabei treten im wesentlichen zwei Probleme auf. Zum einen muß in Anbetracht der geringen Feldstärke der zu messenden Magnetfelder die Sensoranordnung Signale hoher Qualität liefern, die frei von durch Umgebungseinflüsse, z.B. Störfelder, Hochfrequenzfelder oder mechanische Vibrationen, hervorgerufenen Störungen sind. Zum anderen müssen die von der Sensoranordnung stammenden Signale in der Datenverarbeitungsanlage in einer solchen Weise verarbeitet werden, daß eine ausreichend exakte Übereinstimmung der mittels der Datenverarbeitungsanlage gewonnenen Ergebnisse mit den tatsächlichen Verhältnissen vorliegt. Dabei besteht ein Zusammenhang zwischen den beiden Problemen insofern, als nur die Lösung beider Probleme zu mit der Realität ausreichend exakt übereinstimmenden Ergebnissen führen kann.

Die zur Lösung des erstgenannten Problems bisher unternommenen Anstrengungen hatten zumeist die Sensoranordnung und die Abschirmkammer zum Gegenstand.

So wurden z.B. Sensoranordnungen entwickelt, die sehr gut abgeglichene Gradiometer zweiter Ordnung enthalten, die auf 10⁻⁴ oder besser abgeglichen sind, d.h. deren Empfindlichkeit für homogene Felder um den Faktor 10⁴ oder mehr reduziert ist (Druckschrift der Fa. Biomagnetic Technology Inc., San Diego, California: Design and Performance of a 14-Channel Neuromagnetometer", Duane Crum et al., 1985). Die hier beschriebene Sensoranordnung enthält insgesamt 14 Gradiometer zweiter Ordnung, angeordnet in zwei Kryostaten, und zwar in Gruppen von je sieben Gradiometern.

Herstellung und Betrieb einer so gut abgeglichenen Sensoranordnung verursachen erheblichen Aufwand und Kosten. Bereits die Herstellung einzelner Gradiometer zweiter Ordnung mit der genannten geringen Empfindlichkeit für homogene Felder ist mit einem nicht unerheblichen Aufwand verbunden. Bei Anordnung und Betrieb in einem Array von Gradiometern zweiter Ordnung sind zusätzliche Maßnahmen zur Reduzierung wechselseitiger elektrischer und mechanischer Störungen zwischen den einzelnen Gradiometern und zwischen den Gradiometern und deren Halterung zu treffen. Der Aufwand für die in der genannten Druckschrift beschriebene Meßeinrichtung wird dadurch weiter erhöht, daß Maßnahmen zur Messung magnetischer Störfelder und zur elektronischen Kompensation der gemessenen Störfelder getroffen sind.

Weiter wurde eine in siebenschaliger Bauweise ausgeführte Abschirmkammer mit sehr hoher Schirmwirkung für Magnetfelder entwickelt, deren innere Schale aus Kupfer gebildet ist, während die übrigen Schalen aus Mumetall bestehen (A. Mager: "The Berlin Magnetically Shielded Room (BMSR)" in Biomagnetism, Walter de Gruyter & Co., Berlin-New York, 1981, Seite 73 ff.). Dieser Aufbau führt einerseits zu einer sehr hohen Abschirmwirkung von 3 x 10⁴ bei 0,5 Hz, 3 x 10⁵ bei 5 Hz, 1,5 x 10⁵ bei 50 Hz und 10⁶ bei hohen Frequenzen und ermöglicht dadurch die Verwendung von unkompensierten Meßspulen, wird jedoch andererseits durch extremen Aufwand der Konstruktion und entsprechende Kosten erkauft.

Eine weitere Abschirmkammer mit sehr hoher Schirmwirkung ist bei V. Kelhä: "Construction and Performance of the Otaniemi Magnetically Shielded Room", in Biomagnetism, Walter de Gruyter & Co., Berlin New York, 1981, Seiten 33 bis 50, beschrieben. Mit dieser Abschirmkammer, die drei Mumetallschalen, die jeweils zwischen zwei Aluminiumschichten eingeschlossen sind, eine geregelte aktive Abschirmung sowie weitere Abschirmmaßnahmen auf Grundlage des sogenannten Shaking-Verfahrens aufweist, werden vergleichbar hohe Schirmfaktoren erreicht, jedoch ist auch hier der Aufwand für den Aufbau von insgesamt neun Schalen und den Betrieb der Kammer erheblich. Zusätzlich ergibt sich ein die Qualität der Signale beeinträchtigender Effekt dadurch, daß die innerste Schicht der Abschirmkammer aus Aluminium besteht und so störende Einflüsse durch Wirbelströme in der Aluminiumschale auftreten.

Eine Einrichtung der eingangs genannten Art ist aus dem Artikel "Large-area low-noise seven-channel dc SQUID magnetometer for brain research" von Knuutila et al., erschienen in Review of Scientific Instrument 58(11), November 1987, S. 2145-2156 in Verbindung mit dem Artikel "Design, Construction, and Performance of a Large-Volume Magentic Shield" von Kelhä et al. erschienden in IEEE Transactions on Magnetics, Vol MAG-18, No. 1, Jan. 1982, Seiten 260-270 beschrieben. Ein Array mit sieben Gradiometern erster Ordnung ist in einer dreiwandigen magnetischen Abschirmkammer angeordnet, wobei jede Wand der Abschirmkammer aus zwei gekreuzten Schichten Mumetall, die zwischen Aluminiumplatten eingepreßt sind, besteht. Diese neunlagige Abschirmkammer weist sehr hohe Schirmfaktoren auf, so ist z.B. bei magnetischen Wechselfeldern mit einer Frequenz von 0,1 Hz der Schirmfaktor größer als 10⁴ und oberhalb von 0,5 Hz größer als 10⁵. Jedoch lassen sich aus einer einzigen Messung nur grobe Übersichtsfeldlinienbilder erzeugen, während detailierte Feldlinienbilder nur mit mehreren Messungen erzeugt werden können, bei denen die Positionen des Arrays unterschiedlich sind. Zusätzlich muß in jeder Meßposition mehrmals gemessen werden, wobei die Meßwerte gemittelt werden. Trotz des hohen Abschirmaufwandes ist mit einer derartigen Einrichtung die Messung und Lokalisierung von spontanen, d.h. unvorhergesehenen, elektrophysiologischen Aktivitäten nicht möglich.

Die Sensoranordnungen und Abschirmkammern nach dem Stand der Technik gestatten es demgemäß nicht, eine Einrichtung der eingangs genannten Art zu realisieren, die in der Lage ist, Meßsignale der erforderlichen Qualität zu liefern.

Ein Betriebsverfahren für Einrichtungen der eingangs genannten Art ist in der US-PS 4 736 751 in seinen Grundzügen beschrieben. Dieses Verfahren setzt eine Sensoranordnung mit wenigstens 32 Gradiometern voraus, jedoch werden keine Angaben dazu gemacht, welche Qualität die Signale der Gradiometer für das bekannte Verfahren besitzen müssen und mit welcher Einrichtung Signale in hinreichender Qualität gewonnen werden können.

Der Erfindung liegt die Aufgabe zugrunde, eine Einrichtung der eingangs genannten Art so auszubilden, daß mit möglichst geringem apparativen Aufwand Signale erhalten werden, die die Gewähr dafür bieten, daß ihre Verarbeitung mittels einer elektronischen Datenverarbeitungsanlage zu brauchbaren Ergebnissen führt. Außerdem liegt der Erfindung die Aufgabe zugrunde, ein Verfahren zum Betrieb einer Einrichtung der eingangs genannten Art anzugeben, das es gestattet, ausgehend von mit möglichst geringem apparativen Aufwand erhaltenen Signalen der Realität möglichst exakt entsprechende Ergebnisse zu erhalten.

Gemäß der Erfindung wird die die Einrichtung betreffende Aufgabe in einer ersten Variante dadurch gelöst, daß
h) die Abschirmkammer zweischalig aufgebaut ist, wobei die innere Schale aus einem weichmagnetischem Werkstoff gebildet ist, dessen relative Permeabilität größer als 10⁴ ist, und die äußere Schale aus einem unmagnetischen, elektrisch gut leitendem Material besteht.

Gemäß der Erfindung wird die die Einrichtung betreffende Aufgabe in einer zweiten Variante dadurch gelöst, daß
h) die Abschirmkammer dreischalig aufgebaut ist, wobei die innere und die äußere Schale jeweils aus Mumetall gebildet sind, während die mittlere Schale aus Aluminium gebildet ist.

Überraschenderweise hat sich gezeigt, daß eine derartige Einrichtung trotz der Verwendung von Gradiometern erster Ordnung in der Sensoranordnung und trotz der Verwendung einer Abschirmkammer, deren Abschirmfaktor erheblich unterhalb des Abschirmfaktors üblicherweise verwendeter Abschirmkammern liegt, unter der Voraussetzung, daß die Sensoranordnung ein Array von wenigstens zehn Gradiometern erster Ordnung aufweist, Signale von solch hoher Qualität liefert, daß die Voraussetzungen dafür geschaffen sind, der Realität sehr gut entsprechende Ergebnisse zu erhalten. Die erfindungsgemäße Einrichtung kann mit vergleichsweise geringem Aufwand realisiert werden, da Gradiometer erster Ordnung im Vergleich zu Gradiometern höherer Ordnung sehr einfach herstellbar sind und im Falle der erfindungsgemäßen Einrichtung ein genauer Abgleich der Gradiometer entfallen kann. So genügt es, wenn die Gradiometer für homogene Magnetfelder auf weniger als 2 x 10⁻² abgeglichen sind. Dies ist erreichbar, ohne daß bei der Herstellung der Gradiometer besondere Maßnahmen getroffen werden müssen. Auch der Aufwand für die Abschirmkammer ist im Falle der erfindungsgemäßen Einrichtung vergleichsweise gering, da gemäß der Erfindung keine extrem hohe Abschirmwirkung erforderlich ist. Für hochfrequente Wechselfelder sollte die Abschirmkammer zweckmäßigerweise einen Abschirmfaktor (Feldstärke außerhalb der Abschirmkammer bezogen auf die Feldstärke innerhalb der Abschirmkammer) in der Größenordnung von 1 000 aufweisen. Die gemäß der Erfindung vorgesehenen an sich bekannten DC-SQUIDs unterscheiden sich von ebenfalls bekannten RF-SQUIDs dadurch, daß DC-SQUIDs ein geringeres Eigenrauschen aufweisen als RF-SQUIDs.

Zwar ist bei M.S. Hämäläinen et al.: "Characterization of Brain Noise with a 7-Channel SQUID Magnetometer" in Japanese Journal of Applied Physics, Vol. 26 (1987), Supplement 26-3, Seiten 1569 bis 1570, eine Sensoranordnung mit sieben Gradiometern erster Ordnung und einer entsprechenen Anzahl von DC-SQUIDs beschrieben. Wie Untersuchungen gezeigt haben, erfordern Systeme mit sieben Kanälen in einer zusammenhängenden Sensorfläche die serielle Aufnahme der Signale in verschiedenen Positionen der Sensoranordnung, damit eine Quelle erfaßt werden kann. Notwendig ist jedoch ein System, welches die simultane Aufnahme aller für eine Quellenerfassung notwendigen Signale erlaubt. Bei Vorhandensein von Rauschanteilen im Signal ist die dafür notwendige Mindestzahl der Kanäle zehn.

Gemäß einer besonders vorteilhaften Variante der Erfindung enthält die Sensoranordnung wenigstens zwölf Gradiometer erster Ordnung, bei denen es sich vorzugsweise ausschließlich um Gradiometer der gleichen Bauart handelt. Diese Maßnahmen gestatten es, der Realität sehr gut entsprechende Meßergebnisse unter Anwendung eines Rechenverfahrens vergleichsweise geringer Komplexität zu erhalten, da eine Erhöhung der Zahl der Gradiometer um zwei die Genauigkeit der Ergebnisse noch merklich steigert.

Eine Variante der Erfindung sieht vor, daß die Abschirmkammer derart ausgebildet ist, daß die Inhomogenität des im Inneren der Abschirmkammer während einer Messung vorliegenden magnetischen Restfeldes kleiner als 100 nT/m ist. Diese Inhomogenität des Restfeldes und die angegebenen Abschirmfaktoren können erreicht werden, wenn die Abschirmkammer zweischalig aufgebaut ist, wobei die innere Schale aus Mumetall gebildet sind, während die mittlere Schale aus Aluminium gebildet ist. Eine derartige Abschirmkammer bietet außerdem den Vorteil, daß die innere Schale aus Mumetall das magnetische Rauschen, das durch Wirbelströme in der mittleren aus Aluminium gebildeten Schale erzeugt wird, zu dem das Untersuchungsobjekt und die Sensoranordnung enthaltenden Innenraum der Abschirmkammer hin abschirmt. Außerdem besitzt eine derartige Abschirmkammer für hochfrequente Wechselfelder (Frequenz größer als 1 kHz) einen Abschirmfaktor von wenigstens 1 000. Die Abschirmkammer kann gemäß einer Ausführungsform der Erfindung mit einer Entmagnetisierungseinrichtung versehen sein, die die Abschirmkammer mit einem Entmagnetisierungsfeld beaufschlagt, das zur Entmagnetisierung der Abschirmkammer über mindestens vier Größenordnungen, ausgehend von der Sättigungsfeldstärke für Mumetall, reduzierbar ist. Durch diese Maßnahme werden die Flußdichte und die Inhomogenität des im Inneren der Abschirmkammer vorhandenen Restfeldes weiter reduziert. Eine besonders vorteilhafte Ausführung ergibt sich, wenn die Abschirmkammer dreischalig aufgebaut ist, wobei zusätzlich zur zweischaligen Ausführung eine weitere äußere Mumetall-Schale angebracht ist.

Eine Variante der Erfindung sieht vor, daß die Sensoranordnung und die Lagerungseinrichtung derart gehaltert sind, daß die zwischen der Sensoranordnung und der Lagerungseinrichtung infolge von mechanischer Anregung während einer Messung auftretenden Abstandsänderungen kleiner sind als 100 µm. Dabei ist der Begriff mechanische Anregung so zu verstehen, daß er alle Einflüsse umfaßt, die zu unerwünschten Abstandsänderungen zwischen der Sensoranordnung und der Lagerungseinrichtung führen können, also z.B. Erschütterungen des Bodens oder Schallwellen. Nur äußerst geringe Abstandsänderungen infolge mechanischer Anregung treten zwischen der Sensoranordnung und der Lagerungseinrichtung dann auf, wenn gemäß einer Variante der Erfindung ein Fundament für die Abschirmkammer vorgesehen ist, auf dem die Halterung der Sensoranordnung und die Lagerungseinrichtung getrennt voneinander angebracht sind. Dabei ist das Fundament vorzugsweise auf einem Sandbett gelagert und eine direkte mechanische Verbindung sowohl zwischen der Halterung der Sensoranordnung und den Schalen der Abschirmkammer als auch der Lagerungseinrichtung und den Schalen der Abschirmkammer vermieden.

Die Gradiometer sind gemäß einer bevorzugten Ausführungsform als axiale Gradiometer ausgebildet, deren Emfindlichkeit für homogene Magnetfelder geringer als etwa 2 x 10⁻² ist. Unter einem axialen Gradiometer versteht man ein Gradiometer, dessen Feld- und Kompensationsspule eine gemeinsame Mittelachse aufweisen. Die Verwendung axialer Gradiometer bietet den Vorteil, daß eine höhere Zahl von Gradiometern in einer vorgegebenen Fläche angeordnet werden kann als z.B. bei planaren Gradiometern, bei denen Feld- und Kompensationsspule nebeneinander in einer Fläche liegen. Außerdem ist die Signalintensität entsprechend höher.

Die Feldspulen der Gradiometer umschließen gemäß einer Variante der Erfindung jeweils eine vorzugsweise etwa kreisförmige Fläche von wenigstens 3,5 cm². Dabei kann vorgesehen sein, daß die die Feldspulen der Gradiometer enthaltende Sensorfläche etwa kreisförmig ausgebildet ist und einen Durchmesser von wenigstens 8 cm Durchmesser aufweist. Durch die genannten Maßnahmen wird der Vorteil erzielt, daß eine zur flächendeckenden Erfassung einer Quelle ausreichende Meßfläche verfügbar ist und die notwendige Mindestzahl von Gradiometern darin angeordnet werden kann.

Nach einer Variante der Erfindung ist vorgesehen, daß die Verstärkeranordnung einen Vorverstärker aufweist, dem ein Lockin-Verstärker nachgeschaltet ist, wobei die Anzahl der Vorverstärker und der Lockin-Verstärker jeweils der Anzahl von Verstärkerkanälen der Verstärkeranordnung entspricht.

In einer besonders vorteilhaften Ausführung ist in jedem Verstärkerkanal ein Isolationsverstärker entweder zwischen den Vorverstärker und den Lockin-Verstärker geschaltet oder dem Lockin-Verstärker nachgeschaltet. Der Isolationsverstärker dient dazu, eine Potentialtrennung zwischen den Verstärkern vorzunehmen. Dadurch werden Masseschleifen vermieden.

Jedem Verstärkerkanal kann ein auf die Frequenz des Netzes und deren ganzzahlige Vielfache abgestimmtes Kammfilter nachgeschaltet sein, um entsprechende Störungen aus den Signalen ausfiltern zu können. Außerdem kann zwischen dem Isolationsverstärker und dem Analog/Digital-Wandler in jedem Verstärkerkanal ein Antialiazing-Filter vorgesehen sein, um das Auftreten von Artefakten bei der Analog/Digital-Wandlung zu vermeiden.

Wenn als Untersuchungsobjekt ein Lebewesen vorgesehen ist, können zusätzlich zur Messung magnetischer Signale mit der Datenverarbeitungsanlage verbundene Mittel zur Messung wenigstens einer physiologischen Funktion, z.B. der Herztätigkeit (EKG), des als Untersuchungsobjekt vorgesehenen Lebewesens vorhanden sein. Es besteht so die Möglichkeit, die erhaltenen Ergebnisse in Abhängigkeit von der gemessenen physiologischen Funktion zu interpretieren. Außerdem können mit der Datenverarbeitungsanlage verbundene Mittel zur Stimulierung der Sinne des als Untersuchungsobjekt vorgesehenen Lebewesens vorhanden sein, um die Auswirkungen einer Stimulierung der Sinne des Lebewesens auf die Ergebnisse beurteilen zu können.

Eine bevorzugte Variante der Erfindung sieht vor, daß Mittel zur Positionierung des Untersuchungsobjektes auf der Lagerungseinrichtung in einer definierten Lage in bezug auf die Lagerungseinrichtung und Mittel zur Bestimmung der räumlichen Lage der Sensoranordnung relativ zu der Lagerungseinrichtung vorgesehen sind. Anhand der entsprechenden Daten ist es möglich, mittels der Datenverarbeitungsanlage die räumliche Lage der Sensoranordnung in bezug auf das Untersuchungsobjekt zu be stimmen, was für den Erhalt exakter Ergebnisse von besonderer Wichtigkeit ist.

Der dem Betriebsverfahren einer Einrichtung der eingangs genannten Art betreffende Teil der Aufgabe wird durch ein Verfahren gelöst, bei dem
a) über einen Meßzeitraum die von den Gradiometern stammenden Signale als Funktion der Zeit in digitalisierter Form in der Datenverarbeitungsanlage gespeichert werden,
b) für den Meßzeitraum mittels der Datenverarbeitungsanlage anhand der von den Mitteln zur Bestimmung der räumlichen Lage der Sensoranordnung relativ zu der Lagerungseinrichtung stammenden Daten Daten bezüglich der räumlichen Lage der Sensoranordnung relativ zu dem Untersuchungsobjekt ermittelt und als Funktion der Zeit in digitalisierter Form in der Datenverarbeitungsanlage gespeichert werden,
c) unter Zugrundelegung eines Modells des Untersuchungsobjektes mittels der Datenverarbeitungsanlage anhand der von den Gradiometern von gleichen Zeitpunkten innerhalb des Meßzeitraums stammenden Signale und der die räumliche Lage der Sensoranordnung relativ zu dem Untersuchungsobjekt betreffenden Daten für eine Quelle magnetischer Signale wahlweise
   i. der zeitliche Verlauf der magnetischen Flußdichte der Quelle,
   ii. der zeitliche Verlauf der magnetischen Flußdichte der Quelle in einer der Anordnung der Gradiometer entsprechenden Darstellung,
   iii. für einen vorgegebenen Zeitpunkt in dem Meßzeitraum eine Feldlinienkarte, in der für eine frei definierbare Ebene zu der Quelle gehörige Linien gleicher magnetischer Flußdichte enthalten sind, oder
   iv. für einen vorgegebenen Zeitpunkt in dem Meßzeitraum die räumliche Lage der Quelle
   berechnet wird und
d) die Meßergebnisse über die Ausgabeeinrichtung ausgegeben werden.

Mittels des erfindungsgemäßen Verfahrens ist es möglich, ausgehend von den Signalen einer Einrichtung mit nur zehn, vorzugsweise zwölf, Gradiometern der Realität weitgehend entsprechende Ergebnisse zu erhalten. Dabei kann der mittels der Datenverarbeitungsanlage vorzunehmenden Berechnung als Modell des Untersuchungsobjektes eine Kugel oder ein Halbraum homogener Leitfähigkeit zugrundegelegt werden. Die mittels der Datenverarbeitungsanlage vorgenommenen Berechnungen beruhen darauf, daß bei Lebewesen bevorzugte Untersuchungsgebiete Hirn und Herz sind, wobei das Hirn als leitfähige Kugel und das Herz samt Thorax als leitfähiger Halbraum, in welchem sich jeweils die Quellen magnetischer Signale als Stromdipol befinden, modelliert werden.

Die Ausgabe der Ergebnisse in Form des zeitlichen Verlaufes der magnetischen Flußdichte der Quelle bietet sich insbesondere dann an, wenn aus Kurvenform und Zeitverlauf einerseits die Qualität der Signale beurteilt werden soll und andererseits den herkömmlichen elektrischen Verfahren (EEG, EKG) entsprechende diagnostische Aussagen gewonnen werden sollen. Dagegen ist die Ausgabe der Ergebnisse in Form von Feldlinienkarten dann besonders vorteilhaft, wenn die flächenhafte Verteilung der Signale, insbesondere im Hinblick auf eine Auswahl von einer weitergehenden Auswertung zugänglichen Abschnitten, betrachtet wird sowie zur Darstellung der Dynamik der physiologischen Vorgänge. Die Ausgabe der Lage der räumlichen Quelle als Ergebnis wird man dann wählen, wenn aus der Untersuchung Schlüsse hinsichtlich des raum-zeitlichen Verlaufes von Körperfunktionen oder sogar für daraus abgeleitete Therapiemaßnahmen zu ziehen sind. Beispiele sind die Erfassung der Erregungsherde bei fokaler Epilepsie des Hirns oder die Reizleitung des Herzens.

Eine bevorzugte Ausführungsform des erfindungsgemäßen Verfahrens sieht vor, daß in der Datenverarbeitungsanlage ein mittels eines Schnittbildgerätes, z.B. mittels eines Magnetresonanz-Tomographen, angefertigtes anatomisches Bild des Untersuchungsobjektes gespeichert wird und daß die Ausgabe der Ergebnisse derart erfolgt, daß das anatomisches Bild, vorzugsweise ein dreidimensionales Bild, des Untersuchungsobjektes dargestellt wird, in das die Lage der Quelle für einen vorgegebenen Zeitpunkt des Meßzeitraumes eingetragen ist. Dieser Art der Ausgabe der Ergebnisse ist besonders anschaulich und informativ. Dabei kann vorgesehen sein, daß eine Folge von vorzugsweise dreidimensionalen anatomischen Bildern, in die jeweils wenigstens die räumliche Lage der Quelle eingetragen ist, für aufeinanderfolgende Zeitpunkte innerhalb des Meßzeitraumes ausgegeben wird.

Wenn als Untersuchungsobjekt ein Lebewesen vorgesehen ist, kann gemäß Varianten der Erfindung vorgesehen sein, daß während des Meßzeitraumes die Sinne des Lebewesens stimuliert werden, z.B. optisch und/oder akustisch, und die Ausgabe von Ergebnissen für Zeitpunkte innerhalb des Meßzeitraumes erfolgt, in denen die Sinne des Lebewesens stimuliert werden. Außerdem kann vorgesehen sein, daß während des Meßzeitraumes eine periodische physiologische Funktion des Lebewesens gemessen und als Funktion der Zeit in digitalisierter Form in der Datenverarbeitungsanlage gespeichert wird. Es ist dann gemäß einer Variante der Erfindung möglich, daß die Ausgabe von Ergebnissen für Zeitpunkte erfolgt, in denen die physiologische Funktion des Lebewesens einen definierten Wert besitzt. Es ist so möglich, die erhaltenen Ergebnisse in Abhängigkeit von einer Stimulation der Sinne des Lebewesens bzw. einer physiologischen Funktion des Lebewesens zu beurteilen.

Ein Ausführungsbeispiel der Erfindung ist in den beigefügten Figuren dargestellt. Es zeigen:
- Fig. 1: die wesentlichen mechanischen Komponenten einer erfindungsgemäßen Einrichtung in teilweise geschnittener Darstellung,
- Fig. 2: eine Seitenansicht der Halterung für die Sensoranordnung,
- Fig. 3: eine Aufsicht auf die Halterung gemäß Fig. 2,
- Fig. 4: in schematischer Darstellung den Aufbau der Sensoranordnung,
- Fig. 5: die Anordnung der in der Sensoranordnung gemäß Fig. 4 enthaltenen axialen Gradiometer in der Sensorfläche,
- Fig. 6: in schematischer Darstellung die erfindungsgemäße Einrichtung gemäß Fig. 1 einschließlich der elektronischen Komponenten,
- Fig. 7: ein Flußdiagramm bezüglich eines Betriebsverfahrens für die Einrichtung nach den Fig. 1 bis 6, und
- Fig. 8 bis 10: Beispiele für mittels der Einrichtung nach den Fig. 1 bis 6 bei der Durchführung des Verfahrens gemäß Fig. 7 erhaltenen Ergebnisse.

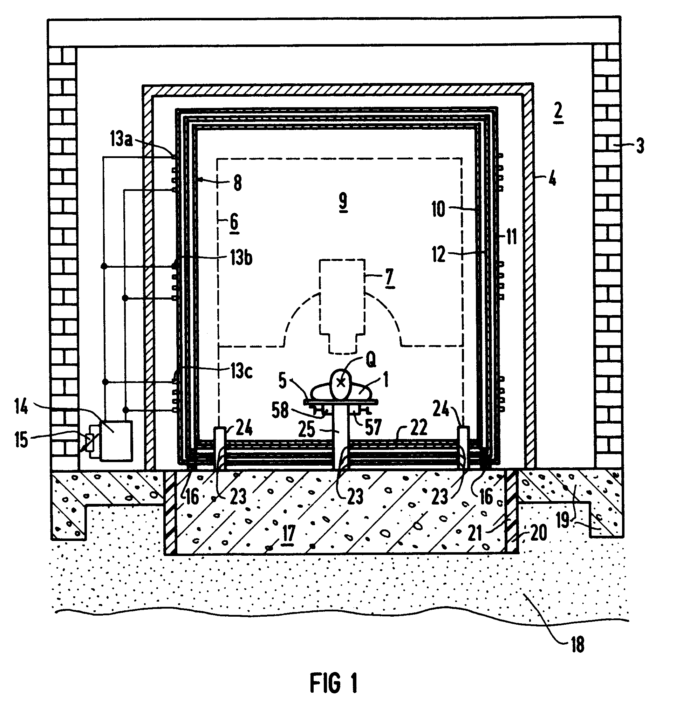

In Fig. 1 ist eine erfindungsgemäße Einrichtung zur Messung von schwachen, orts- und zeitabhängigen biomagnetischen Feldern, die von im Inneren eines als Untersuchungsobjekt vorgesehenen menschlichen Patienten 1 befindlichen Quellen ausgehen. Die Einrichtung ist jedoch auch für Messungen an Untersuchungsobjekten aus toter Materie geeignet. Die erfindungsgemäße Einrichtung, die insgesamt mit dem Bezugszeichen 2 versehen ist, befindet sich in einer Halle 3 und ist von einer Außenwand 4 umgeben. Die Außenwand 4 ist beispielsweise gemauert, aus Rigipsplatten gebildet oder aus schalldämmendem Material gefertigt und dient vornehmlich dazu, die Einrichtung 2 von außen vor Vibration und mechanischer Beschädigung zu schützen. Die Einrichtung 2 weist eine als Patientenliege 5 ausgebildete Lagerungseinrichtung für den Patienten 1, eine über dem Patienten 1 an einer strichliert angedeuteten Halterung 6 aufgehängte, ebenfalls strichliert angedeutete Sensoranordnung 7 auf, die zur Messung von im Inneren des Patienten 1 befindlichen Quellen ausgehenden Magnetfeldern dient. Im Falle der Fig. 1 ist die Sensoranordnung auf eine im Schädel des Patienten 2 befindliche Quelle Q ausgerichtet. Außerdem besitzt die Einrichtung eine die Patientenliege 5 mit dem daraufliegenden Patienten 1 und die Sensoranordnung 7 umschließende etwa kubisch geformte Abschirmkammer 8, durch die der im Inneren der Abschirmkammer 8 befindliche Meßraum 9 magnetisch abgeschirmt ist.

Die Abschirmkammer 8 ist dreischalig ausgeführt, wobei die innere Schale 10 und die äußere Schale 11 jeweils aus Mumetall gebildet sind, während die mittlere Schale 12 aus Aluminium besteht. Die Abschirmkammer 8 weist für magnetische Wechselfelder mit einer Frequenz von 0,5 Hz einen Abschirmfaktor von wenigstens 10 für magnetische Wechselfelder mit einer Frequenz von 5 Hz einen Abschirmfaktor von wenigstens 100 und für magnetische Wechselfelder mit einer Frequenz von 50 Hz und darüber einen Abschirmfaktor von wenigstens 1000 auf. Für hochfrequente Wechselfelder besitzt die Abschirmkammer 8 einen Abschirmfaktor von wenigstens 1 000. Die Inhomogenität des im Meßraum 9 bei normalen Umgebungsbedingungen vorliegenden magnetischen Restfeldes ist kleiner als 100 nT/m. Um die genannten Daten zu erreichen, ist die äußere Schale 11 nicht unbedingt notwendig. Diese kann demgemäß auch entfallen.

Die Abschirmkammer 8 ist von Spulen 13a, 13b, 13c umgeben, die mit einem Wechselstromgenerator 14 verbunden sind und mit diesem eine Entmagnetisierungseinrichtung bilden, mittels derer die Abschirmkammer 8 mit einem Entmagnetisierungsfeld beaufschlagt werden kann, das zur Entmagnetisierung der Abschirmkammer über mindestens vier Größenordnungen, ausgehend von der Sättigungsfeldstärke für Mumetall, kontinuierlich reduzierbar ist, was durch einen mit dem Wechselstromgenerator 14 verbundenen Einstellwiderstand 15 angedeutet ist.

Die Abschirmkammer 8 ist über eine oder mehrere Halterungen 16 starr mit einem Fundamentsockel 17 hoher Masse verbunden. Die Masse des Fundamentsockels 17 sollte in der Größenordnung von 10 bis 20 Tonnen liegen. Er besteht vorzugsweise aus eisenfreiem Beton. Der quaderförmige Fundamentsockel 17 ist auf ein Sandbett 18 gesetzt. Die Halle 3 weist ein Hallenfundament 19 mit einer Bodenplatte auf, die durch einen Zwischenraum 20 mechanisch von dem Fundamentsockel 17 getrennt ist. Der Zwischenraum 20 ist mit einem Kunststoffschaum 21 ausgefüllt. Durch diese Maßnahme ist sichergestellt, daß mechanische Schwingungen von außerhalb der Halle 3, die beispielsweise durch den Straßenverkehr hervorgerufen werden, nicht direkt auf den Fundamentsockel 17 übertragen werden.

Die Abschirmkammer 8 weist eine Bodenplatte 22 auf, die im Bereich des Bodens der Abschirmkammer 8 auf deren innerer Schale 10 angebracht ist. Die Schalen 10, 11, 12 und die Bodenplatte 22 weisen im Bereich des Bodens der Abschirmkammer 8 eine Anzahl von Aussparungen 23 auf, durch welche jeweils ein Pfosten 24 bzw. 25 hindurchgeführt ist. Die Pfosten 24, 25 sind aus einem elektrisch isolierenden Material, beispielsweise Holz, Keramik oder Kunststoff, gefertigt. Sie sind mit ihrem unteren Ende jeweils fest an dem Fundamentsockel 17 verankert. Auf einer ersten Anzahl von Pfosten, nämlich auf den Pfosten 24, ist die Halterung 6 für die Sensoranordnung 7 befestigt. Auf einer zweiten Anzahl von Pfosten, nämlich auf den Pfosten 25, von denen nur einer sichtbar ist, ist die Patientenliege 5 befestigt.

Bewegungen des auf der Patientenliege 5 liegenden Patienten 1, müssen also erst über die Pfosten 25 auf den Fundamentsockel 17 geleitet werden, bevor sie von dort über die Pfosten 24 auf die Halterung 6 und von dort auf die Sensoranordnung 7 übertragen werden können. Dies gilt umgekehrt für etwaige Vibrationen der Sensoranordnung 7. Aufgrund der großen Masse des Fundamentsockels 17 sind somit Bewegungen der an der Halterung 6 angebrachten Sensoranordnung 7 relativ zu der Patientenliege 5 bzw. dem daraufliegenden Patienten 1 weitgehend unterdrückt. Ebenso sind Schwingungen, die durch einen auf der Bodenplatte 22 laufenden Arzt angeregt werden, nur über die Masse des Fundamentsockels 17 auf die Sensoranordnung 7 bzw. die Patientenliege 5 übertragbar. Falls eine Übertragung von Schwingungen der Bodenplatte 22 auf die Abschirmkammer 8 unerwünscht ist, kann die Bodenplatte 22 in nicht dargestellter Weise ebenfalls über weitere Pfosten direkt mit dem Fundamentsockel 17 verbunden werden.

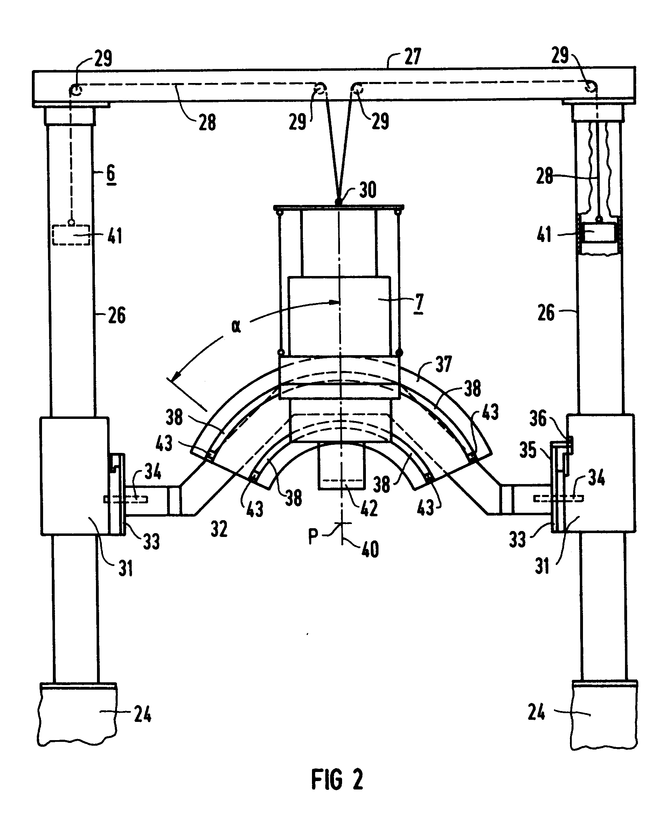

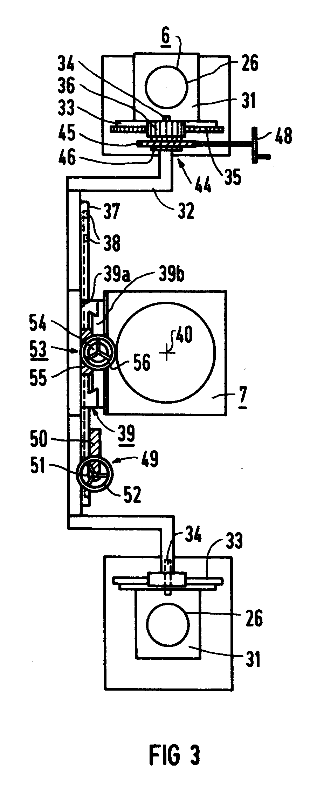

Wenn für die Sensoranordnung 7 eine Halterung gemäß den Figuren 2 und 3 vorgesehen ist, sind die infolge von mechanischer Anregung zwischen der Sensoranordnung 7 und der Patientenliege 5 bei einer Messung auftretenden Abstandsänderungen kleiner als 100 µm. Abstandsänderungen dieser Größe führen in einem Restfeld mit einer Inhomogenität von 100 nT/m zu Feldänderungen von höchstens 2 x 10⁻¹³T, unter der Voraussetzung, daß die Sensoranordnung 7 Gradiometer enthält, die auf 2 x 10⁻² abgeglichen sind. Diese Feldänderungen liegen unterhalb der Signalstärke der meisten biomagnetischen Signale.

In den Fig. 2 und 3 ist die Halterung 6 für die Sensoranordnung 7 gezeigt, die zwei vertikale Stativsäulen 26 besitzt, deren untere Enden mit den Pfosten 24 fest verbunden sind und die an ihren oberen Enden über eine Querstrebe 27 miteinander verbunden sind. Die Stativsäulen 26 sind innen hohl und dadurch geeignet, jeweils ein innen verschiebbares Gegengewicht 41 aufzunehmen. Jedes Gegengewicht 41 ist über ein Seil 28, welches über Umlenkrollen 29 bis zur Mitte der hohlen Querstrebe 27 läuft, mit einer gemeinsamen Öse 30 verbunden, welche an der Sensoranordnung 7 befestigt ist.

An den Stativsäulen 26 ist jeweils ein Laufkasten 31 höhenverschiebbar angebracht. Die Laufkästen 31 sind über einen Bügel 32 miteinander verbunden. Der Bügel 32 weist an seinen Enden je ein Kreisscheibensegment 33 auf, das drehbar um einen an dem zugeordneten Laufkasten 31 befestigten Bolzen 34 gelagert ist. Am Umfang eines der Kreisscheibensegmente 33 ist ein Zahnkranz 35 vorgesehen, an welchem ein Zahnradantrieb 36 angreift. Der Bügel 32 kann somit um eine durch die Bolzen 34 verlaufende Achse geschwenkt werden.

In der Mitte des Bügels 32 ist ein Kreisringsegment 37 angebracht. Das Kreisringsegment 37 ist mit einer oder bevorzugt mehreren parallelen kreissegmentförmigen Nuten 38 versehen. In den Nuten 38 ist die Sensoranordnung 6 über an ihr befestigte Nutensteine schwenkbar gelagert. Der Schwenkwinkel bezüglich der Vertikalen ist mit "Alpha" bezeichnet. Der Verstellung um den Winkel "Alpha" erfolgt über ein Stirnradgetriebe. Das Kreisringsegment 37 ist beim Schwenken jeweils parallel zur Bügelebene ausgerichtet.

Über einen Schlitten 39 ist die Sensoranordnung 7 in ihrer Höhe relativ zum Kreisringsegment 37 verschiebbar. Die Verschiebung erfolgt dabei immer in radialer Richtung, also in Richtung des Radius des Kreisringsegmentes 37. Über ein Gleitlager (nicht gezeigt) ist die Sensoranordnung 7 weiterhin um ihre Zentralachse 40 drehbar.

Mit Hilfe der zuvor beschriebenen Gegengewichte 41 in Verbindung mit den Seilen 28 und den Umlenkrollen 29 ist die Gesamtheit von Sensoranordnung 7, Kreisringsegment 37, Bügel 32 und Laufkästen 31 in der Höhe, also relativ zu den Stativsäulen 26, verschiebbar. Die Querstrebe 27 dient also neben der mechanischen Versteifung gleichzeitig als Brücke für einen Gewichtsausgleich zwischen dem Gewicht der Sensoranordnung 7 einerseits und den in den Stativsäulen 26 gleitenden Gegengewichten 41 andererseits. Die Höhenverstellung dient dazu, die Sensoranordnung 7 mit der strichliert angedeuteten Sensorfläche 42 dicht an den Patienten 1 heranzubringen.

Entlang der Nuten 38 ist die Sensoranordnung 7 um den Winkel Alpha verschiebbar. Der maximale Winkel Alpha beträgt dabei ca. 50° nach jeder Seite hin. In den Nuten 38 ist endseitig je ein Anschlag 43 vorgesehen, der ein Anstoßen der geschwenkten Sensoranordnung 7 an die Stativsäule 26 verhindert. Sowohl beim Schwenken um den Winkel Alpha als auch beim Drehen des Bügels bleibt die Sensorfläche 42 auf den gleichen Punkt P ausgerichtet. Der Punkt ist in der Regel in den Kopf oder in den Thorax des Patienten 1 an eine Stelle gelegt, in deren Nähe sich eine Quelle Q magnetischer Signale befindet. Mit Hilfe des Schlittens 39 kann der Abstand zwischen der Sensorfläche 42 und dem Punkt P variiert werden.

Aus der Fig. 3 geht hervor, daß dem Zahnradantrieb 36 zumindest einseitig ein Schneckengetriebe 44 zugeordnet ist, das ein Schneckenrad 45, eine Schnecke 46 sowie ein Handrad 48 zur Verstellung umfaßt. Außerdem ist ein Schneckengetriebe 49 (mit Schneckenrad 50, Schnecke 51 und Handrad 52) zwischen Schlitten 39 und Kreisringsegment 37 vorgesehen, mit dessen Hilfe der Schwenkwinkel "Alpha" verstellt und arretiert werden kann. Die Fig. 2 zeigt, daß der Schlitten 39 in zwei Teile 39a und 39b unterteilt ist. Das Teil 39a sitzt in der Nut 38 und das Teil 39b ist relativ zum Teil 39a in der radialen Richtung verschiebbar (z.B. über eine Schwalbenschwanzführung). Der Winkel "Alpha " ergibt sich durch Verschieben des Teiles 39a entlang der Nut 38, wodurch das Teil 39b mitbewegt wird. Zur Radialverschiebung des Teiles 39b relativ zum Teil 39a ist ein weiteres Schneckengetriebe 53 mit dem Schneckenrad 54, der Schnecke 55 und dem Handrad 56 vorhanden.

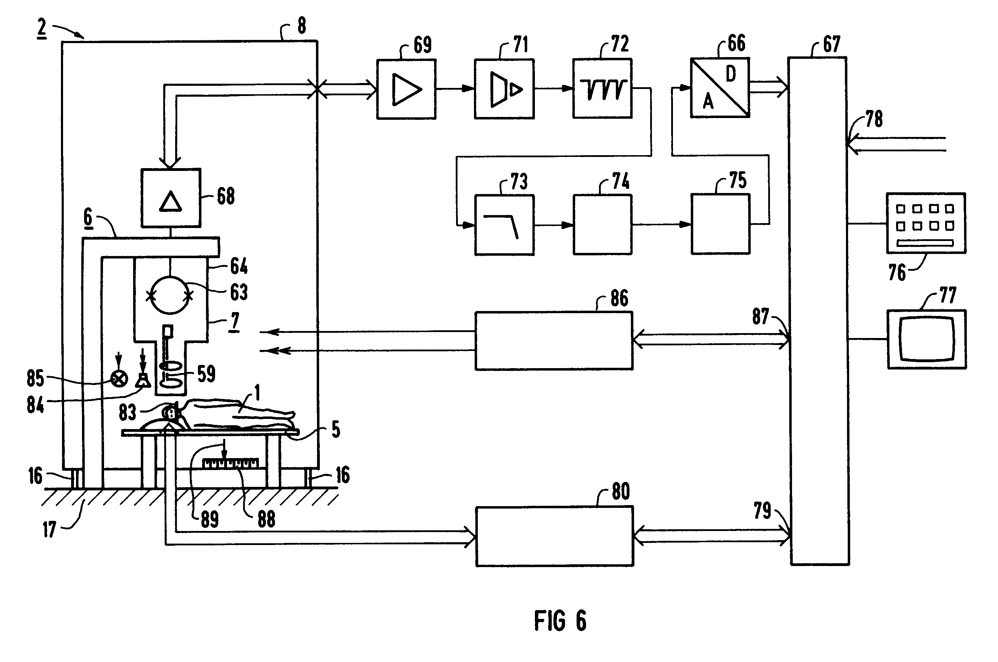

Zusätzlich zu den Einstellmöglichkeiten der Sensoranordnung 7 relativ zu der Patientenliege 5, die sich aus der beschriebenen Ausbildung der Halterung 6 ergeben, ergeben sich weitere Verstellmöglichkeiten der genannten Komponenten relativ zueinander dadurch, daß die Patientenliege 5 in einer parallel zum Boden der Abschirmkammer 8 verlaufenden Ebene mit Hilfe von zwei von Hand betätigbaren Antrieben 57, 58 in Richtung ihrer Längsachse und quer dazu verstellbar ist. Diesbezügliche höhere Einzelheiten sind nicht dargestellt. Geeignete Lösungsmöglichkeiten sind dem Fachmann bekannt. Infolge der beschriebenen Ausbildung der Halterung 6 und der Patientenliege 5 ist es möglich, die Sensoranordnung 7 mit ihrer Sensorfläche 42 auf gewünschte Körperzonen des Patienten 1 auszurichten. Die Position der Patientenliege 5 samt Patient 1 relativ zu der Sensoranordnung 7 kann in an sich bekannter Weise erfaßt werden, z.B. mit einer Anordnung ablesbarer Meßschalen, die in nicht dargestellter Weise den Schneckengetrieben 44, 49, 53 zugeordnet bzw. an der Patientenliege 5 angebracht sind. Eine Skala 88 und ein Zeiger 89 zur Ermittlung der Lage der Patientenliege 5 in deren Längsrichtung in bezug auf die Sensoranordnung 7 in Fig. 6 sind beispielhaft eingezeichnet. Dabei nimmt die Skala 88 eine definierte Lage in bezug auf die Sensoranordnung 7 ein.

Die Halterung 6 und die Patientenliege 5 sind in ihrer Gesamtheit aus anderen als ferromagnetischen Werkstoffen gebildet.

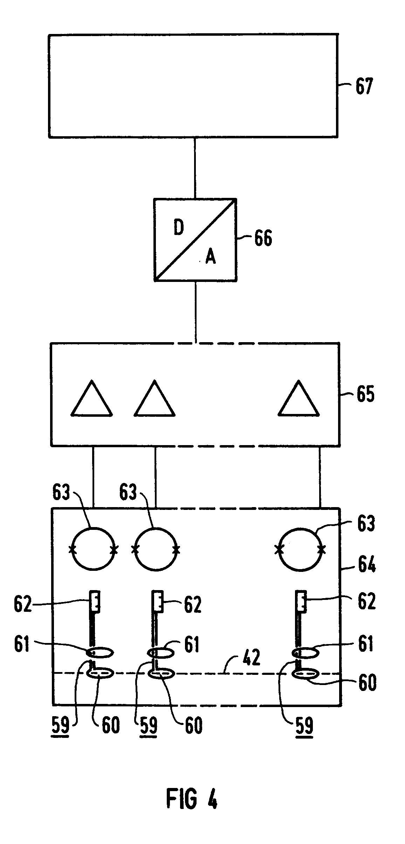

In der Fig. 4 ist stark schematisiert der Aufbau der Sensoranordnung 7 dargestellt. Die Sensoranordnung 7 weist ein Array von zwölf Gradiometern erster Ordnung auf, die jeweils das Bezugszeichen 59 tragen, wobei in Fig. 4 nur einige der Gradiometer 59 dargestellt sind. Bei den Gradiometern 59 handelt es sich um axiale Gradiometer. Diese besitzen in bekannter Weise jeweils eine Feldspule 60 und eine mit dieser verbundene Kompensationsspule 61, wobei die Kompensationsspule 61 jeweils gegensinnig zu der entsprechenden Feldspule 60 gewickelt ist und beide eine gemeinsame Mittelachse aufweisen. Die Kompensationsspule 61 jedes Gradiometers 59 ist in einer Ebene angeordnet, die parallel zu der Ebene der entsprechenden Feldspule 60 verläuft. Für Magnetfelder, die in größerer Entfernung von einem derart aufgebauten Gradiometer entstehen, heben sich die in die Feldspule 60 und die Kompensationsspule 61 induzierten Spannungen entsprechend dem Abgleich auf. Liegt die Quelle eines Magnetfeldes jedoch in der Nähe eines derartigen Gradiometers 59, wie dies für eine in einem in der Nähe der Sensoranordnung 7 befindlichen Untersuchungsobjekt, z.B. dem Patienten 1, vorhandene Quelle der Fall ist, so ist die in der Feldspule 60 induzierte Spannung erheblich größer als die in der Kompensationsspule 61 induzierte Spannung. Dabei wirkt sich vorteilhaft aus, daß im Nahbereich der Quelle die Feldstärke sehr rasch abnimmt. Die im Falle der beschriebenen Sensoranordnung vorgesehenen Gradiometer 59 weisen für homogene Magnetfelder eine um den Faktor 2 x 10⁻² reduzierte Empfindlichkeit auf. Derartige Gradiometer können mit geringem Aufwand realisiert werden.

Die Feldspulen 60 der Gradiometer 59 umschließen jeweils eine Fläche von mehr als 3,5 cm². Sind die Feldspulen 60 wie in Fig. 4 dargestellt von etwa kreisförmiger Gestalt, weisen sie einen Druchmesser von mehr als 2,1 cm auf. Feldspulen 60 und Kompensationsspulen 61 weisen einen Abstand von 7 cm auf.

Die Gradiometer 59 sind jeweils mit einer Spule 62 verbunden, die dazu dient, die Gradiometer jeweils mit einem DC-SQUID eines Arrays von zwölf SQUIDs induktiv zu koppeln. Wie schon der Name sagt, arbeiten die SQUIDs, die den Josephson-Effekt ausnutzen, im supraleitenden Zustand. Die Gradiometer 59 und die SQUID's 63 sind daher in einem schematisch angedeuteten Thermobehälter, einem sogenannten Kryostaten, der das Bezugszeichen 64 trägt, angeordnet. Dieser enthält flüssiges Helium, das bekanntermaßen eine Temperatur von ca. 4,2° K aufweist. Die Feldspulen 60 der Gradiometer 59 sind in einer strichliert angedeuteten Sensorfläche 42 dicht hinter der ihnen gegenüberliegenden Wand des Kryostaten 64 angeordnet. Weitere Einzelheiten bezüglich des Aufbaus von derartigen Sensoranordnungen 7 sind der bereits genannten Veröffentlichung von Duane Crum et al. sowie H. Jablonski: "Zum Stand der kommerziell zur Verfügung stehenden SQUID-Meßsysteme zur Messung biomagnetischer Signale", Ergebnisberichte zum Programm "Forschung und Entwicklung im Dienste der Gesundheit - Biomagnetische Signale / SQUID-Meßsysteme", DFVLR-NT 1 84/1, Seiten 29 bis 42, zu entnehmen.

Die einzelnen SQUIDs 63 des Arrays sind mit einer noch näher zu beschreibenden Verstärkeranordnung 65 verbunden, die Bestandteil einer elektronischen Einrichtung zur Verstärkung und Auswertung der Signale der Gradiometer 59 ist, die außer der Verstärkeranordnung 65 einen mit deren Ausgang verbundenen Analog/Digial-Wandler 66 aufweist, dessen Ausgang mit einer elektronischen Datenverarbeitungsanlage 67 verbunden ist, die u.a. zur Auswertung der von den Gradiometern 59 stammenden Signale dient. Wie in der Fig. 4 angedeutet ist, enthält die Verstärkeranordnung 65 eine der Anzahl der Gradiometer 59 bzw. der SQUID's 63 entsprechende Anzahl von Verstärkerkanälen, von denen jeder mit einem der DC-SQUIDs 63 verbunden ist.

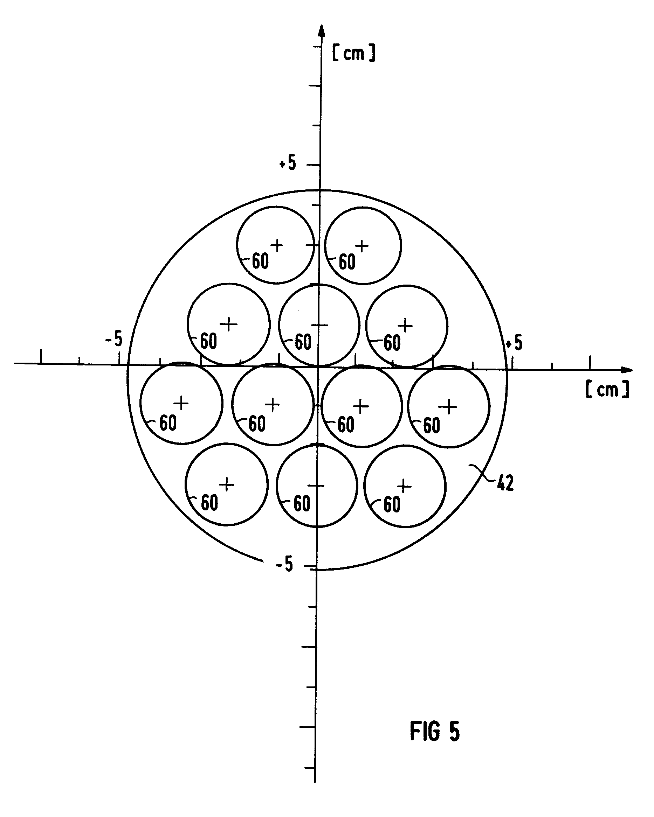

Die Anordnung von zwölf Gradiometern in der Sensorfläche 42 der Sensoranordnung 7 ist in Aufsicht in der Fig. 5 schematisch angedeutet. Gemäß dieser Fig. ergibt sich durch die gewählte Anordnung eine gute Ausnutzung der zur Verfügung stehenden Fläche, die von etwa kreisförmiger Gestalt ist und einen Durchmesser von ca. 10 cm aufweist. Die in Fig. 5 angedeuteten Feldspulen 60 der Gradiometer besitzen jeweils einen Durchmesser von 2,1 cm und umschließen somit eine Fläche von 3,5 cm².

Weitere Einzelheiten bezüglich der Verstärkeranordnung 65 ergeben sich aus der Fig. 6, in der die erfindungsgemäße Einrichtung mit allen wesentlichen mechanischen und elektronischen Komponenten dargestellt ist. In dieser Fig. ist nur ein Gradiometer 59 mit dem zugehörigen DC-SQUID 63 und dem nachfolgenden Verstärkerkanal der Verstärkeranordnung 65 dargestellt. Die übrigen Kanäle sind identisch aufgebaut. Gemäß Fig. 6 weist ein Verstärkerkanal einen im Bereich der Sensoranordnung 7 angeordneten Vorverstärker 68 auf, der mit dem dem jeweiligen Kanal entsprechenden SQUID verbunden ist. Das Ausgangssignal des Vorverstärkers 68 gelangt jeweils zu einem Lockin-Verstärker 69, der das jeweilige Eingangssignal auf konstantem Pegel hält und dessen rückgekoppeltes Signal das Ausgangssignal darstellt. An den Lockin-Verstärker 69 schließt sich jeweils ein Isolationsverstärker 71 an, der eine Potentialtrennung zwischen den Verstärkern vornimmt, um Masseschleifen zu vermeiden.

Dem Isolationsverstärker 71 jedes Verstärkerkanals ist zur Unterdrückung von durch das elektrische Netz verursachten Störungen ein Kammfilter 72 nachgeschaltet, das auf die Frequenz des elektrischen Netzes und deren ganzzahlige Vielfache abgestimmt ist. An das Kammfilter 72 schließt sich jeweils ein steilflankiges Antialiazing-Filter 73 an, von dem die Signale jeweils zu einer Sample and Hold-Schaltung 74 gelangen. Die Sample and Hold-Schaltungen 74 sind über einen 12zu1-Multiplexer 75 mit dem Analog-Eingang des Analog/Digital-Wandlers 66 verbunden, der mit seinem Digital-Ausgang an die Datenverarbeitungsanlage 67 angeschlossen ist. Die Sample and Hold-Schaltungen 74, der 12zu1-Multiplexer 75 und der Analog/Digital-Wandler 66 erhalten die jeweils erforderlichen Steuerungssignale von der Datenverarbeitungsanlage 67. Die Antialiazing-Filter 73 besitzen zur Vermeidung von Artefakten jeweils eine Zeitkonstante, die mindestens der doppelten Wandlungszeit des Analog/Digital-Wandlers 66 multipliziert mit zwölf (Anzahl der Kanäle) entspricht. Grundsätzlich besteht auch die Möglichkeit, für jeden der Verstärkerkanäle der Verstärkeranordnung 65 einen gesonderten Analog/Digital-Wandler vorzusehen. Der Multiplexer 75 kann dann entfallen. Die Zeitkonstanten der Antialiazing-Filter 73 können dann so gewählt werden, daß sie mindestens der doppelten Wandlungszeit der Analog/Digital-Wandler entsprechen. Es ergibt sich somit gegenüber der zuvor beschriebenen Anordnung eine um einen der Kanalzahl entsprechenden Faktor, der im Falle des Ausführungsbeispieles 12 beträgt, höhere Grenzfrequenz.

Die Datenverarbeitungsanlage 67 umfaßt in an sich bekannter und nicht dargestellter Weise eine Zentraleinheit (CPU), einen Programmspeicher und einen Datenspeicher. An die Datenverarbeitungsanlage 67 sind als Eingabeeinrichtung eine Tastatur 76 und als Ausgabeeinrichtung für Ergebnisse der Auswertung der von den Gradiometern 59 stammenden Signale ein Sichtgerät 77 angeschlossen. Als Ausgabeeinrichtung kann außerdem ein Drucker und/oder Plotter vorgesehen sein (nicht dargestellt). Die Datenverarbeitungsanlage 67 besitzt außerdem eine Schnittstelle 78, über die sie mit einem nicht dargestellten Schnittbildgerät, z.B. einem Magnetresonanz-Tomographen, verbunden werden kann, so daß zusätzlich zu den mittels des Analog/Digital-Wandlers 66 aus den Signalen der Sensoranordnung 7 gewonnenen Daten mittels des Schnittfeldgerätes angefertigte vorzugsweise dreidimensionale anatomische Bilder des Untersuchungsobjektes in digitalisierter Form in dem Datenspeicher der Datenverarbeitungsanlage 67 gespeichert werden können.

Weiter besitzt die Datenverarbeitungsanlage 67 eine Schnittstelle 79, an die eine in der Fig. 6 schematisch angedeutete Einrichtung zur Messung wenigstens einer physiologischen Funktion des Patienten 1, z.B. ein an sich bekannters EEG-Gerät 80, angeschlossen ist. Die von dem EEG-Gerät 80 gelieferten Daten werden ebenfalls in digitalisierter Form in dem Datenspeicher der Datenverarbeitungsanlage 67 gespeichert. Der Patient 1 nimmt in Fig. 6 eine Seitenlage ein, wobei sein Schädel durch ein Polster unterstützt ist.

Die Position der Patientenliege 5 relativ zu der Sensoranordnung 7 kann wie erwähnt z.B. durch Ablesen von Skalen erfaßt und mittels der Tastatur 76 in die Datenverarbeitungsanlage 67 eingegeben werden. Eine automatische Erfassung über an sich bekannte elektrische oder optische Positionssensoren einschließlich Übermittlung an die Datenverarbeitungsanlage 67 ist ebenfalls möglich.

Um eine definierte räumliche Lage des Patienten 1 in bezug auf die Patientenliege 5 herstellen zu können, ist ein in Fig. 6 schematisch angedeutetes Aufbißstück 83 vorgesehen, das fest mit der Patientenliege 5 verbunden ist, und auf das der auf der Patientenliege 5 liegende Patient 1 während der Untersuchung beißt. Daten bezüglich der Lage des Aufbißstückes 83 relativ zu der Patientenliege 5 sind in der Datenverarbeitungsanlage 67 gespeichert, so daß diese in der Lage ist, anhand dieser Daten und der die räumliche Lage der Sensoranordnung 7 relativ zu der Patientenliege 5 betreffenden Daten, die räumliche Lage des Schädels des Patienten 1 relativ zu der Sensoranordnung 7 zu ermitteln. Sollen andere Körperteile des Patienten 1 als dessen Schädel untersucht werden, können anstelle des Aufbißstückes 83 andere geeignete Mittel zur Herstellung einer definierten Lage des Patienten 1 in bezug auf die Patientenliege 5 Verwendung finden.

Wie in der Fig. 6 schematisch angedeutet ist, sind im Bereich des Schädels des Patienten 1 Mittel zur Stimulierung von dessen Sinnen vorgesehen. Es handelt sich hierbei um einen Lautsprecher 84 und ein Lichtsignal 85. Beide werden mittels einer Steuerungselektronik 86, die über eine Schnittstelle 87 mit der Datenverarbeitungsanlage 67 verbunden ist, in geeigneter Weise zur akustischen und/oder optischen Stimulierung der Sinne des Patienten 1 betätigt.

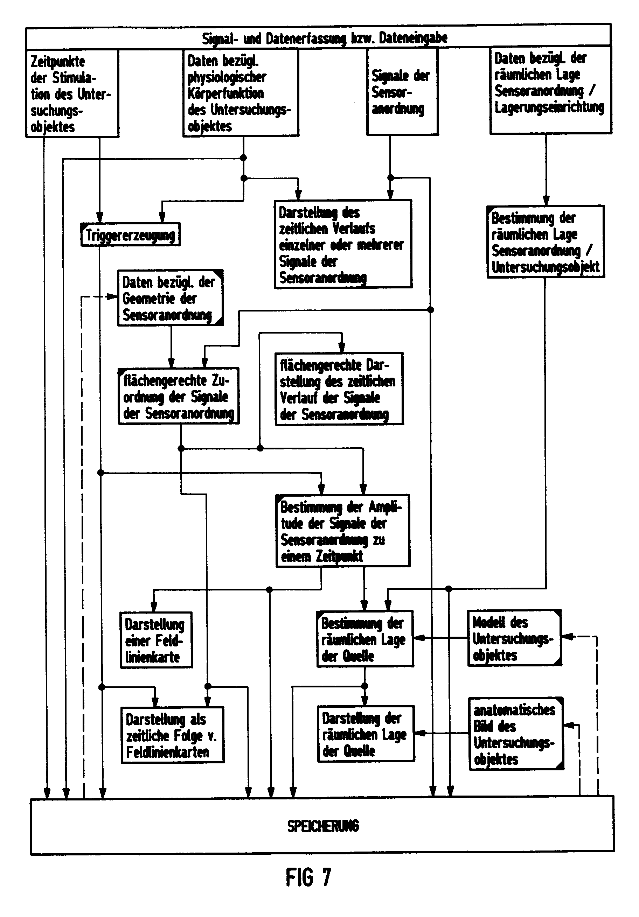

Ein Verfahren zum Betrieb der Einrichtung gemäß den Fig. 1 bis 6 ist in der Fig. 7 in Form eines Flußdiagrammes dargestellt. Demnach werden die von der Sensoranordnung 7 stammenden Signale, die die physiologische Funktion des Patienten 1 betreffenden Daten und Daten, die die Zeitpunkte angeben, zu denen eine Stimulation des Patienten 1 erfolgt, in der Datenverarbeitungsanlage 67 erfaßt sowie Daten bezüglich der Position der Sensoranordnung 7 und der Patientenliege 5 relativ zueinander in die Datenverarbeitungsanlage 67 eingegeben bzw. bei automatischen Sensoren erfaßt. Die von der Sensoranordnung 7 stammenden Signale sowie die die physiologische Funktion des Patienten 1 betreffenden Daten werden in ihrem zeitlichen Verlauf einzeln bzw. für mehrere Kanäle der Sensoranordnung 7 dargestellt, und erforderlichenfalls nach flächengerechter Zuordnung mit Hilfe von in der Datenverarbeitungsanlage 67 gespeicherten Daten bezüglich der Geometrie der Sensoranordnung 7 den Positionen der einzelnen Gradiometer 59 zugeordnet und dargestellt. Aus diesen Ergebnissen können, gegebenenfalls unter zusätzlicher Verwendung der Daten bezüglich der Zeitpunkte, zu denen eine Stimulation des Patienten 1 erfolgt, weitere Signalparameter, wie z.B. die Amplitude, abgeleitet und als Feldlinienkarte dargestellt werden. Unter Einbeziehung der mittels der Datenverarbeitungsanlage 67 ermittelten Daten bezüglich der räumlichen Lage des Patienten 1 relativ zu der Sensoranordnung 7 und eines gespeicherten Modells des untersuchten Körperbereiches des Patienten 1 kann außerdem die räumliche Lage der Quelle Q ermittelt und in Verbindung mit einem durch ein bildgebendes Verfahren gewonnenen und in der Datenverarbeitungsanlage 67 gespeicherten anatomischen Bild des untersuchten Körperbereiches des Patienten 1 dargestellt werden.

Die mittels der Datenverarbeitungsanlage 67 erfaßten Signale und Daten sowie daraus abgeleitete Signale und Daten, die in Fig. 7 dadurch gekennzeichnet sind, daß die entsprechenden Angaben im Flußdiagramm mit einem geschwärzten Dreieck gekennzeichnet sind, können in einem der Datenverarbeitungsanlage 67 zugeordneten Speicher, z.B. Magnetplatte oder dergleichen, gespeichert werden.

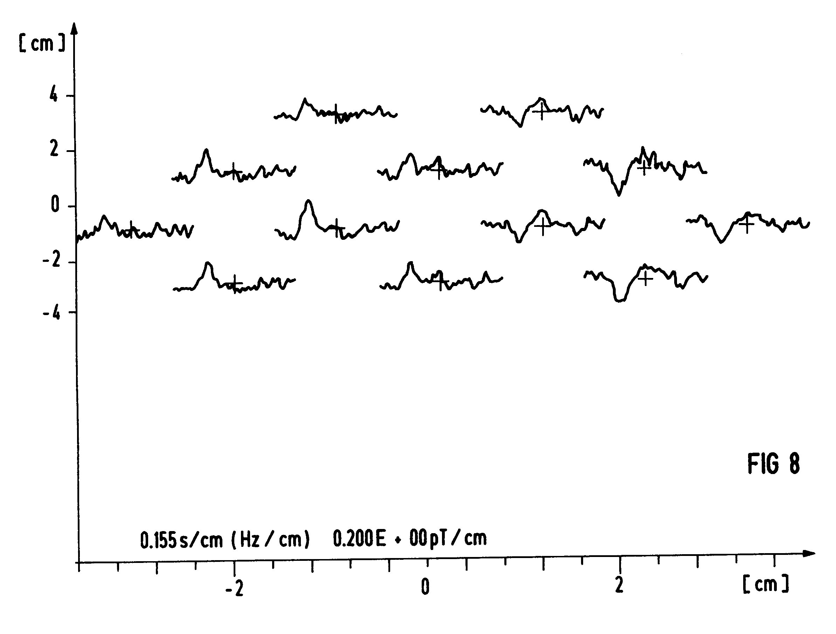

In den Fig. 8 bis 10 sind Meßergebnisse dargestellt, wie sie mit der Einrichtung nach den Fig. 1 bis 6 erhalten werden, wenn diese gemäß dem zuvor erläuterten Verfahren betrieben wird.

Im einzelnen zeigt die Fig. 8 den zeitlichen Verlauf der magnetischen Flußdichte einer Quelle. Dabei sind die zeitlichen Verläufe der Flußdichte, wie sie mit den einzelnen Gradiometern 59 einer Anordnung gemäß Fig. 5 erhalten werden, getrennt dargestellt. Die entsprechenden Kurven sind in Fig. 8 in einer Anordnung relativ zueinander dargestellt, die der Anordnung der Gradiometer 59 gemäß Fig. 5 entspricht.

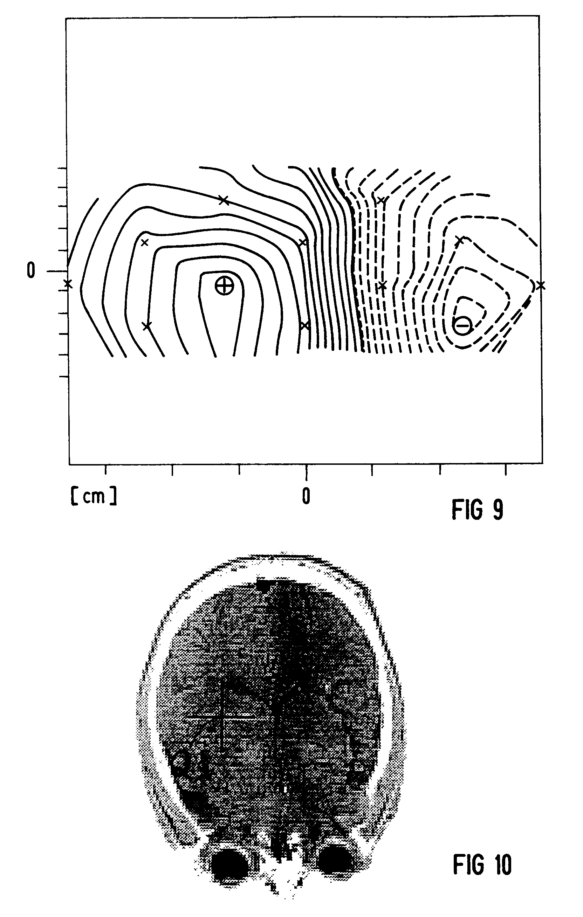

Die Fig. 9 zeigt eine Feldlinienkarte, in die für eine Ebene, die eine definierte Lage in bezug auf eine Quelle Q einnimmt, Linien gleicher magnetischer Flußdichte eingetragen sind. Dabei gibt die Feldlinienkarte die Verhältnisse für einen definierten Zeitpunkt innerhalb des Meßzeitraumes wieder.

Fig. 10 zeigt schließlich ein mittels Magnetresonanz-Tomographie gewonnenes Schnittbild eines Untersuchungsobjektes, nämlich des Schädels eines menschlichen Patienten, in das die Lage einer Quelle Q eingetragen ist. Es handelt sich dabei um ein zweidimensionales Bild. Es besteht jedoch auch die Möglichkeit in dreidimensionale Bilder eines Untersuchungsobjektes die ermittelte räumliche Lage einer Quelle Q einzutragen.

## Patentansprüche

1. Einrichtung zur Messung von schwachen, orts- und zeitabhängigen Magnetfeldern, die von einer im Inneren eines Untersuchungsojektes (1) befindlichen Quelle (Q) ausgehen, welche Einrichtung aufweist:
a) eine Lagerungseinrichtung (5) zur Aufnahme des Untersuchungsobjektes (1),
b) eine Sensoranordnung (7) aufweisend ein Array mit wenigstens zehn Gradiometern erster Ordnung (59), wobei jedes Gradiometer (59) eine Feldspule (60) und eine entsprechende Kompensationsspule (61) aufweist und die Feldspulen (60) der Gradiometer (59) in einer Sensorfläche (42) angeordnet sind, ein Array von einer der Anzahl der Gradiometer (59) entsprechenden Anzahl von DC-SQUIDs (63), wobei jedes Gradiometer (59) mit einem der DC-SQUIDs (63) induktiv gekoppelt ist, und ein die Gradiometer (59) und die entsprechenden DC-SQUIDs (63) enthaltendes Gefäß (64), in dem eine Temperatur herrscht, bei der die DC-SQUIDs (63) und die Gradiometer (59) supraleitend sind,
c) eine Halterung (6) für die Sensoranordnung (7),
d) Mittel (48, 52, 56, 57, 58) zur Verstellung der Lagerungseinrichtung (5) und der Sensoranordnung (7) relativ zueinander derart, daß die Sensoranordnung (7) auf gewünschte Zonen des Untersuchungsobjektes (1) ausrichtbar ist,
e) eine die Lagerungseinrichtung (5) und die Sensoranordnung (7) umschließende Kammer (8) zur Abschirmung magnetischer Felder, wobei
f) die magnetische Abschirmkammer (8) für magnetische Wechselfelder mit einer Frequenz von 0,5 Hz einen Abschirmfaktor von wenigstens 10, für magnetische Wechselfelder mit einer Frequenz von 5 Hz einen Abschirmfaktor von wenigstens 100 und für magnetische Wechselfelder mit einer Frequenz von 50 Hz und darüber einen Abschirmfaktor von wenigstens 1 000 aufweist, und
g) eine elektronische Einrichtung zur Verstärkung und Auswertung der Signale der Gradiometer (59), welche eine mit den DC-SQUIDs (63) verbundene Verstärkeranordnung (65), einen mit der Verstärkeranordnung (65) verbundenen Analog/Digital-Wandler (66) und eine mit diesem verbundene elektronische Datenverarbeitungsanlage (67) aufweist, wobei die Verstärkeranordnung (65) eine der Anzahl der Gradiometer (59) entsprechende Anzahl von Verstärkerkanälen besitzt, von denen jeder mit einem der DC-SQUIDs (63) verbunden ist, und die Datenverarbeitungsanlage (67) eine Ausgabeeinrichtung (77) für Ergebnisse der Auswertung der Signale der Gradiometer (59) aufweist,
**dadurch gekennzeichnet,**
h) die Abschirmkammer (8) zweischalig aufgebaut ist, wobei eine innere Schale aus einem weichmagnetischem Werkstoff gebildet ist, dessen relative Permeabilität größer als 10⁴ ist, und eine äußere Schale aus Aluminium besteht.

2. Einrichtung nach dem Oberbegriff des Anspruchs 1, **dadurch gekennzeichnet,** daß
h) die Abschirmkammer (8) dreischalig aufgebaut ist, wobei die innere und die äußere Schale (10 bzw. 11) jeweils aus Mumetall gebildet sind, während die mittlere Schale (12) aus Aluminium gebildet ist.

3. Einrichtung nach Anspruch 1 oder 2, **dadurch gekennzeichnet,** daß die Sensoranordnung (7) wenigstens zwölf Gradiometer erster Ordnung (59) enthält.

4. Einrichtung nach einem der Ansprüche 1 bis 3, **dadurch gekennzeichnet,** daß die Sensoranordnung (7) ausschließlich Gradiometer (59) der gleichen Bauart enthält.

5. Einrichtung nach einem der Ansprüche 1 bis 4, **dadurch gekennzeichnet,** daß die Inhomogenität des im Inneren der Abschirmkammer (8) während einer Messung vorliegenden magnetischen Restfeldes kleiner als 100 nT/m ist.

6. Einrichtung nach einem der Ansprüche 1 bis 5, **dadurch gekennzeichnet,** daß die Abschirmkammer (8) mit einer Entmagnetisierungs-Einrichtung (13a,13b,13c,14,15) verbunden ist, die die Abschirmkammer (8) mit einem Entmagnetisierungsfeld beaufschlagt, das zur Entmagnetisierung der Abschirmkammer (8) über mindestens vier Größenordnungen kontinuierlich reduzierbar ist.

7. Einrichtung nach einem der Ansprüche 1 bis 6, **dadurch gekennzeichnet,** daß die Sensoranordnung (7) und die Lagerungseinrichtung (5) derart gehaltert sind, daß zwischen der Sensoranordnung (7) und der Lagerungseinrichtung (5) infolge von mechanischer Anregung während einer Messung auftretenden Abstandsänderungen kleiner sind als 100 µm.

8. Einrichtung nach einem der Ansprüche 1 bis 9, **dadurch gekennzeichnet,** daß ein Fundament (17) für die Abschirmkammer (8) vorgesehen ist, auf dem die Halterung (6) der Sensoranordnung (7) und die Lagerungseinrichtung (5) getrennt voneinander angebracht sind.

9. Einrichtung nach einem der Ansprüche 1 bis 8, **dadurch gekennzeichnet,** daß die Gradiometer (59) als axiale Gradiometer ausgebildet sind, die für homogene Magnetfelder besser als 2 x 10⁻² abgeglichen sind.

10. Einrichtung nach einem der Ansprüche 1 bis 9, **dadurch gekennzeichnet,** daß die Feldspulen (60) der Gradiometer (59) jeweils eine Fläche von wenigstens 3,5 cm² umschließen.

11. Einrichtung nach einem der Ansprüche 1 bis 10, **dadurch gekennzeichnet,** daß die die Feldspulen (60) der Gradiometer (59) enthaltende Sensorfläche (42) etwa kreisförmig ausgebildet ist und einen Durchmesser von wenigstens 8 cm aufweist.

12. Einrichtung nach einem der Ansprüche 1 bis 11, **dadurch gekennzeichnet,** daß die Verstärkeranordnung (65) einen Vorverstärker (68) aufweist, dem ein Lockin-Verstärker (69) nachgeschaltet ist, wobei die Anzahl der Vorverstärker (68), der Lockin-Verstärker (69) jeweils der Anzahl von Verstärkerkanälen entspricht.

13. Einrichtung nach Anspruch 12, **dadurch gekennzeichnet,** daß in jedem Verstärkerkanal ein Isolationsverstärker (71) entweder zwischen den Vorverstärker (68) und den Lockin-Verstärker (69) geschaltet oder dem Lockin-Verstärker (69) nachgeschaltet ist.

14. Einrichtung nach Anspruch 13, **dadurch gekennzeichnet,** daß jedem Verstärkerkanal ein auf die Frequenz des elektrischen Netzes und deren ganzzahlige Vielfache abgestimmtes Kammfilter (72) nachgeschaltet ist.

15. Einrichtung nach einem der Ansprüche 1 bis 13, **dadurch gekennzeichnet,** daß zwischen der Verstärkeranordnung (65) und dem Analog/Digital-Wandler (66) in jedem Verstärkerkanal ein Antialiazing-Filter (73) vorgesehen ist.

16. Einrichtung nach einem der Ansprüche 1 bis 15, **dadurch gekennzeichnet,** daß mit der Datenverarbeitungsanlage (67) verbundene Mittel (80) zur Messung wenigstens einer physiologischen Funktion eines als Untersuchungsobjekt (1) vorgesehenen Lebewesens vorhanden sind.

17. Einrichtung nach einem der Ansprüche 1 bis 16, **dadurch gekennzeichnet,** daß mit der Datenverarbeitungsanlage (67) verbundene Mittel (84, 85) zur Stimulierung der Sinne eines als Untersuchungsobjekt (1) vorgesehenen Lebewesens vorhanden sind.

18. Einrichtung nach einem der Ansprüche 1 bis 17, **dadurch gekennzeichnet,** daß Mittel (83) zur Positionierung des Untersuchungsobjektes (1) auf der Lagerungseinrichtung (5) in einer definierten Lage in bezug auf die Lagerungseinrichtung (5) und Mittel (88, 89) zur Bestimmung der räumlichen Lage der Sensoranordnung (7) relativ zu der Lagerungseinrichtung (5) vorgesehen sind.

19. Betriebsverfahren einer Einrichtung nach Anspruch 18, bei den
a) über einen Meßzeitraum die von den Gradiometern (59) stammenden Signale als Funktion der Zeit in digitalisierter Form in der Datenverarbeitungsanlage (67) gespeichert werden,
b) für den Meßzeitraum mittels der Datenverarbeitungsanlage (67) anhand der von den Mitteln (88, 89) zur Bestimmung der räumlichen Lage der Sensoranordnung (7) relativ zu der Lagerungseinrichtung (5) stammenden Daten Daten bezüglich der räumlichen Lage der Sensoranordnung (7) relativ zu dem Untersuchungsobjekt (1) ermittelt und als Funktion der Zeit in digitalisierter Form in der Datenverarbeitungsanlage (67) gespeichert werden,
c) unter Zugrundelegung eines Modells des Untersuchungsobjektes (1) mittels der Datenverarbeitungsanlage (67) anhand der von den Gradiometern (59) von gleichen Zeitpunkten innerhalb des Meßzeitraums stammenden Signale und der die räumliche Lage der Sensoranordnung (7) relativ zu dem Untersuchungsobjekt (1) betreffenden Daten für eine Quelle (Q) magnetischer Signale wahlweise
i. der zeitliche Verlauf der magnetischen Flußdichte der Quelle (Q),
ii. der zeitliche Verlauf der magnetischen Flußdichte der Quelle (Q) in einer der Anordnung der Gradiometer entsprechenden Darstellung,
iii. für einen vorgegebenen Zeitpunkt in dem Meßzeitraum eine Feldlinienkarte, in der für eine frei definierbare Ebene zu der Quelle (Q) gehörige Linien gleicher magnetischer Flußdichte enthalten sind, oder
iv. für einen vorgegebenen Zeitpunkt in dem Meßzeitraum die räumliche Lage der Quelle (Q) berechnet wird und
d) die Ergebnisse über die Ausgabeeinrichtung (77) ausgegeben werden.

20. Verfahren nach Anspruch 19, **dadurch gekennzeichnet,** daß der mittels der Datenverarbeitungsanlage (67) vorzunehmenden Berechnung eine Kugel homogener Leitfähigkeit als Modell des Untersuchungsobjektes (1) zugrundegelegt wird.

21. Verfahren nach Anspruch 19, **dadurch gekennzeichnet,** daß der mittels der Datenverarbeitungsanlage (67) vorzunehmenden Berechnung ein Halbraum homogener Leitfähigkeit als Modell des Untersuchungsobjektes (1) zugrundegelegt wird.

22. Verfahren nach einem der Ansprüche 19 bis 21, **dadurch gekennzeichnet,** daß für aufeinanderfolgende Zeitpunkte innerhalb des Meßzeitraumes eine Folge von Feldlinienkarten ausgegeben wird.

23. Verfahren nach einem der Ansprüche 19 bis 21, **dadurch gekennzeichnet,** daß in der Datenverarbeitungsanlage (67) ein mittels eines Schnittbildgerätes angefertigtes anatomisches Bild des Untersuchungsobjektes (1) gespeichert wird und daß die Ausgabe der Ergebnisse derart erfolgt, daß das anatomische Bild des Untersuchungsobjektes (1) dargestellt wird, in das die Lage der Quelle (Q) für einen vorgegebenen Zeitpunkt des Meßzeitraumes eingetragen ist.

24. Verfahren nach Anspruch 23, **dadurch gekennzeichnet,** daß eine Folge von anatomischen Bildern mit eingetragener Lage der Quelle (Q) für aufeinanderfolgende Zeitpunkte innerhalb des Meßzeitraumes ausgegeben wird.

25. Verfahren nach einem der Ansprüche 19 bis 24, **dadurch gekennzeichnet,** daß als Untersuchungsobjekt (1) ein Lebewesen vorgesehen ist.

26. Verfahren nach Anspruch 25, **dadurch gekennzeichnet,** daß während des Meßzeitraumes die Sinne des Lebewesens stimuliert werden.

27. Verfahren nach Anspruch 26, **dadurch gekennzeichnet,** daß die Sinne des Lebewesens optisch stimuliert werden.

28. Verfahren nach Anspruch 26, **dadurch gekennzeichnet,** daß die Sinne des Lebewesens akustisch stimuliert werden.

29. Verfahren nach einem der Ansprüche 26 bis 28, **dadurch gekennzeichnet,** daß die Ausgabe von Ergebnissen für Zeitpunkte innerhalb des Meßzeitraumes erfolgt, in denen die Sinne des Lebewesens stimuliert werden.

30. Verfahren nach einem der Ansprüche 25 bis 29, **dadurch gekennzeichnet,** daß während des Meßzeitraumes eine periodische physiologische Funktion des Lebewesens gemessen und als Funktion der Zeit in digitalisierter Form in der Datenverarbeitungsanlage (67) gespeichert wird.

31. Verfahren nach Anspruch 30, **dadurch gekennzeichnet,** daß die Ausgabe von Ergebnissen für Zeitpunkte erfolgt, in denen die physiologische Funktion des Lebewesens einen definierten Wert besitzt.

## Claims

1. Device for measuring weak magnetic fields, variable with location and time, which are emitted from a source (Q) located inside an examination subject (1), which device has:
a) a support device (5) for receiving the examination subject (1),
b) a sensor arrangement (7) having an array of at least ten first order gradiometers (59), each gradiometer (59) having a field coil (60) and a corresponding compensation coil (61), and the field coils (60) of the gradiometers (59) being arranged in a sensor surface (42), an array of DC-SQUIDs (63) corresponding in number to the number of gradiometers (59), each gradiometer (59) being coupled inductively to one of the DC-SQUIDs (63), and a vessel (64) containing the gradiometers (59) and the corresponding DC-SQUIDs (63), this vessel having a temperature therein at which the DC-SQUIDs (63) and the gradiometers (59) are superconducting,
c) a mounting (6) for the sensor arrangement (7),
d) means (48, 52, 56, 57, 58) for adjusting the support device (5) and the sensor arrangement (7) relative to each other so that the sensor arrangement (7) can be oriented towards desired zones of the examination subject (1),
e) a chamber (8) surrounding the support device (5) and the sensor arrangement (7) for shielding from magnetic fields, whereby
f) the magnetic shielding chamber (8) has a shielding factor of at least 10 for magnetic alternating fields having a frequency of 0.5 Hz, a shielding factor of at least 100 for magnetic alternating fields having a frequency of 5 Hz and a shielding factor of at least 1,000 for magnetic alternating fields having a frequency of 50 Hz and above, and
g) an electronic device for amplifying and evaluating signals from the gradiometers (59), which device has an amplifier arrangement (65) connected to the DC-SQUIDs (63), an analog-to-digital converter (66) connected to the amplifier arrangement (65) and an electronic data processing system (67) connected to the analog-to-digital converter (66), the amplifier arrangement (65) having a number of amplifier channels which corresponds to the number of gradiometers (59), each of these amplifier channels being connected to one of the DC-SQUIDs (63), and the data processing system (67) having an output device (77) for results of the evaluation of the signals of the gradiometers (59),
characterised in that
h) the shielding chamber (8) has a double-shell construction, whereby an inner shell is formed from a weak magnetic material having a relative permeability greater than 10⁴ and an outer shell consists of aluminium.

2. Device according to the preamble of claim 1, characterised in that
h) the shielding chamber (8) has a triple-shell construction, whereby the inner shell and the outer shell (10 and 11 respectively) are formed from mumetal, while the middle shell (12) is formed from aluminium.

3. Device according to claim 1 or 2, characterised in that the sensor arrangement (7) comprises at least twelve first order gradiometers (59).

4. Device according to one of claims 1 to 3, characterised in that the sensor arrangement comprises exclusively gradiometers (59) of the same design.

5. Device according to one of claims 1 to 4, characterised in that the inhomogeneity of the residual magnetic field present inside the shielding chamber (8) during measurement is less than 100 nT/m.

6. Device according to one of claims 1 to 5, characterised in that the shielding chamber (8) is connected to a demagnetizing device (13a, 13b, 13c, 14, 15) which applies a demagnetization field to the shielding chamber (8), which demagnetization field can be continuously reduced over a range of at least four orders of magnitude for demagnetization of the shielding chamber (8).

7. Device according to one of claims 1 to 6, characterised in that the sensor arrangement (7) and the support device (5) are mounted so that distance changes occurring between the sensor arrangement (7) and the support device (5) as a result of mechanical excitation during measurement are smaller than 100 µm.

8. Device according to one of claims 1 to 9 (sic), characterised in that a foundation (17) is provided for the shielding chamber (8), with the mounting (6) for the sensor arrangement (7) and the support device (5) being attached to this foundation (17) separately from each other.

9. Device according to one of claims 1 to 8, characterised in that the gradiometers (59) are formed as axial gradiometers which are adjusted to better than 2 x 10⁻² for homogeneous magnetic fields.

10. Device according to one of claims 1 to 9, characterised in that the field coils (60) of the gradiometers (59) each enclose a surface of at least 3.5 cm².

11. Device according to one of claims 1 to 10, characterised in that the sensor surface (42) comprising the field coils (60) of the gradiometers (59) is formed in a substantially circular shape and has a diameter of at least 8 cm.

12. Device according to one of claims 1 to 11, characterised in that the amplifier arrangement (65) has a pre-amplifier (68) to which a lock-in amplifier (69) is connected downstream, with the number of pre-amplifiers (68) and lock-in amplifiers (69) corresponding respectively to the number of amplifier channels.

13. Device according to claim 12, characterised in that, in each amplifier channel, an insulation amplifier (71) is either connected between the pre-amplifier (68) and the lock-in amplifier (69) or is connected downstream to the lock-in amplifier (69).

14. Device according to claim 13, characterised in that a comb filter (72) tuned to the frequency of the electrical network and to whole-number multiples thereof is connected downstream to each amplifier channel.

15. Device according to one of claims 1 to 13, characterised in that an anti-aliasing filter (73) is provided between the amplifier arrangement (65) and the analog-to-digital converter (66) in each amplifier channel.

16. Device according to claims 1 to 15, characterised in that means (80) connected to the data processing system (67) are present for measuring at least one physiological function of a living being provided as the examination subject (1).

17. Device according to one of claims 1 to 16, characterised in that there are means (84, 85) connected to the data processing system (67) for stimulating the senses of a living being provided as the examination subject (1).

18. Device according to one of claims 1 to 17, characterised in that means (83) are provided for positioning the examination subject (1) on the support device (5) in a defined position relative to the support device (5) and means (88, 89) are provided for determining the spatial position of the sensor arrangement (7) relative to the support device (5).

19. Method of operation for a device according to claim 18, in which
a) over a measured period of time, the signals from the gradiometers (59) are stored as a function of time in digitized form in the data processing system (67),
b) data relating to the spatial position of the sensor arrangement (7) relative to the examination subject (1) are determined for the measured period of time by means of the data processing system (67) with reference to the data from the means (88, 89) for determining the spatial position of the sensor arrangement (7) relative to the support device (5), and are stored as a function of time in digitized form in the data processing system (67),
c) on the basis of a model of the examination subject (1), there is selectively calculated by means of the data processing system (67) with reference to the signals from the gradiometers (59) of identical times within the measured period of time and the data concerning the spatial position of the sensor arrangement (7) relative to the examination subject (1), for a source (Q) of magnetic signals:
i. the time characteristic of the magnetic flux density of the source (Q),
ii. the time characteristic of the magnetic flux density of the source (Q) in a representation corresponding to the arrangement of gradiometers,
iii. a flux diagram for a pre-determined time within the measured time period, which diagram comprises lines of equal magnetic flux density associated with the source (Q) for a freely definable plane, or
iv. the spatial position of the source (Q) for a pre-determined time within the measured time period, and
d) the results are displayed by means of the output device (77).

20. Method according to claim 19, characterised in that a sphere of homogeneous conductivity is used as a model of the examination subject (1) for the basis of the calculation to be made by means of the data processing system (67).

21. Method according to claim 19, characterised in that a half-space of homogeneous conductivity is used as a model of the examination subject (1) for the basis of the calculation to be made by means of the data processing system (67).

22. Method according to one of claims 19 to 21, characterised in that a series of flux diagrams for consecutive times within the measured time period are displayed.

23. Method according to one of claims 19 to 21, characterised in that an anatomical image of the examination subject (1) produced by means of a sectional imaging device is stored in the data processing system (67) and in that the output of the results takes place in such a way that the anatomical image of the examination subject (1) is displayed with the insertion into the said image of the position of the source (Q) for a pre-determined time of the measured time period.

24. Method according to claim 23, characterised in that there is displayed a series of anatomical images with the position of the source (Q) inserted therein for consecutive times within the measured time period.

25. Method according to one of claims 19 to 24, characterised in that a living being is provided as the examination subject (1).

26. Method according to claim 25, characterised in that the senses of the living being are stimulated during the measured time period.

27. Method according to claim 26, characterised in that the senses of the living being are stimulated optically.

28. Method according to claim 26, characterised in that the senses of the living being are stimulated acoustically.

29. Method according to one of claims 26 to 28, characterised in that the results are displayed for times within the measured time period at which the senses of the living being are stimulated.

30. Method according to one of claims 25 to 29, characterised in that a periodic physiological function of the living being is measured during the measured time period and is stored as a function of time in digitized form in the data processing system (67).

31. Method according to claim 30, characterised in that results are displayed for times at which the physiological function of the living being has a defined value.

## Revendications

1. Dispositif pour mesurer des champs magnétiques faibles, qui sont fonction du lieu et du temps et sont émis par une source (Q) située à l'intérieur d'un objet d'examen (1), lequel dispositif comprend :
a) un dispositif de support (5) servant à recevoir l'objet d'examen (1),
b) un dispositif de détection (7) possédant un réseau comprenant au moins dix gradiomètres du premier ordre (59), dans lequel chaque gradiomètre (59) possède une bobine inductrice (60) et une bobine de compensation correspondante (61) et des bobines inductrices (60) des gradiomètres (59) sont disposées dans une surface de détection (42), un réseau comprenant un nombre de dispostifs SQUID à courant continu (63), qui correspond au nombre des gradiomètres (59), chaque gradiomètre (59) étant accouplé inductivement à l'un des dispositifs SQUID à courant continu (63), et un récipient (64), qui contient les gradiomètres (59) et les dispositifs SQUID à courant continu correspondants (63) et dans lequel règne une température pour laquelle les dispositifs SQUID à courant continu (63) et les gradiomètres (59) sont supraconducteurs,
c) un support (6) pour le dispositif de détection (7),
d) des moyens (48,52,56,57,58) pour déplacer l'un par rapport à l'autre le dispositif de support (50) et le dispositif de détection (7) de telle sorte que le dispositif de détection (7) puisse être aligné sur des zones désirées de l'objet d'examen (1),
e) une chambre (8) qui enveloppe le dispositif de support (5) et le dispositif de détection (7) et sert à réaliser un blindage vis-à-vis de champs magnétiques,
f) la chambre de blindage magnétique (8) possédant un facteur de blindage égal au moins à 10 pour des champs magnétiques alternatifs d'une fréquence de 0,5 Hz, un facteur de blindage égal au moins à 100 pour des champs magnétiques alternatifs possédant une fréquence de 5 Hz, et un facteur de blindage égal au moins à 1000 pour des champs magnétiques alternatifs possédant une fréquence de 50 Hz et plus,
g) un dispositif électronique servant à amplifier et évaluer les signaux des gradiomètres (50) et qui comporte un dispositif amplificateur (65) raccordé aux dispositifs SQUID à courant continu (63), un convertisseur analogique/numérique (66) raccordé au dispositif amplificateur (65), et une installation électronique de traitement des données (67) raccordée à ce convertisseur, le dispositif amplificateur (65) possédant un nombre de canaux amplificateurs, qui correspond au nombre des gradiomètres (59), et dont chacun est raccordé à l'un des dispositifs SQUID à courant continu (63), tandis que l'installation (67) de traitement de données comporte un dispositif de sortie (77) pour les résultats de l'évaluation des signaux des gradiomètres (59),
caractérisé par le fait que
h) la chambre de blindage (a) est constituée de deux coques, à savoir une coque extérieure réalisée en un matériau magnétique doux, dont la perméabilité relative est supérieure à 10⁴, et une coque extérieure en aluminium.

2. Dispositif selon le préambule de la revendication 1, caractérisé en ce que
h) la chambre de blindage (8) est formée de trois coques, les coques intérieure et extérieure (10 et 11) étant formées respectivement de mumétal, tandis que la coque médiane (12) est réalisée en aluminium.

3. Dispositif suivant la revendication 1 ou 2, caractérisé par le fait que le dispositif de détection (7) cmporte au moins douze gradiomètres du premier ordre (59).

4. Dispositif suivant l'une des revendications 1 à 3, caractérisé par le fait que le dispositif de détection (7) comporte exclusivement des gradiomètres (59) de même type.

5. Dispositif suivant l'une des revendications 1 à 4, caractérisé par le fait que l'hétérogénéité du champ magnétique résiduel présent à l'intérieur de la chambre de blindage (8) pendant une mesure, est inférieure à 100 nT/m.

6. Dispositif suivant l'une des revendications 1 à 5, caractérisé par le fait que la chambre de blindage (8) est raccordée à un dispositif de désaimantation (13a,13b,13c,14,15), qui charge la chambre de blindage (8) avec un champ de désaimantation qui peut être réduit continûment sur au moins quatre ordres de grandeur pour désaimanter la chambre de blindage.

7. Dispositif suivant l'une des revendications 1 à 6, caractérisé par le fait que le dispositif de détection (7) et le dispositif de support (5) sont fixés de telle sorte que des variations de distance qui apparaissent entre le dispositif de détection (7) et le dispositif de support (5) sous l'effet d'une excitation mécanique lors d'une mesure, sont inférieures à 100 µm.

8. Dispositif suivant l'une des revendications 1 à 9, caractérisé par le fait qu'il est prévu, pour la chambre de blindage (8), un socle (17), sur lequel le dispositif de fixation du dispositif de détection (7) et le dispositif de support (5) sont montés séparément l'un de l'autre.

9. Dispositif suivant l'une des revendications 1 à 8, caractérisé par le fait que les gradiomètres (59) sont réalisés sous la forme de gradiomètres axiaux qui sont compensés à une valeur meilleure que 2 x 10⁻² pour des champs magnétiques homogènes.

10. Dispositif suivant l'une des revendications 1 à 9, caractérisé par le fait que les bobines inductrices (60) des gradiomètres (59) enserrent respectivement une surface d'au moins 3,5 cm².

11. Dispositif suivant l'une des revendications 1 à 10, caractérisé par le fait que la surface de détection (42), qui comporte les bobines inductrices (60) des gradiomètres (59), possède une forme approximativement circulaire et un diamètre égal à au moins 8 cm.

12. Dispositif suivant l'une des revendications 1 à 11, caractérisé par le fait que le dispositif amplificateur (65) comporte un préamplificateur (68), en aval duquel est branché un amplificateur de verrouillage (69), le nombre des préamplificateurs (68) et des amplificateurs de verrouillage (69) correspondant respectivement au nombre des canaux amplificateurs.

13. Dispositif suivant la revendication 12, caractérisé par le fait que dans chaque canal amplificateur, un amplificateur d'isolement (71) est branché entre le préamplificateur (68) et l'amplificateur de verrouillage (69) ou est branché en aval de l'amplificateur de verrouillage (69).

14. Dispositif suivant la revendication 13, caractérisé par le fait qu'en aval de chaque canal amplificateur est branché un filtre en peigne (72) qui est réglé sur la fréquence du réseau électrique et sur les multiples entiers de cette fréquence.

15. Dispositif suivant l'une des revendications 1 à 13, caractérisé par le fait qu'un filtre (73) de suppression des images fantômes est branché entre le dispositif amplificateur (65) et le convertisseur analogique/numérique (66), dans chaque canal amplificateur.

16. Dispositif suivant l'une des revendications 1 à 15, caractérisé par le fait que des moyens (80), qui sont raccordés à l'installation de traitement de données (65), sont présents pour la mesure d'au moins une fonction physiologique d'un être vivant prévu en tant qu'objet d'examen (1).

17. Dispositif suivant l'une des revendications 1 à 16, caractérisé par le fait que des moyens (84,85) raccordés à l'installation de traitement de données (67) sont prévus pour simuler les sens d'un être vivant prévu en tant qu'objet d'examen (1).

18. Dispositif suivant l'une des revendications 1 à 17, caractérisé par le fait qu'il est prévu des moyens (83) pour positionner l'objet d'examen (1) sur le dispositif de support (5) dans une position définie par rapport au dispositif de support (5), et des moyens (88,89) pour déterminer la position spatiale du dispositif de détection (16) par rapport au dispositif de support (5).

19. Procédé d'utilisation d'un dispositif suivant la revendication 18, selon lequel
a) pendant un intervalle de temps de mesure, les signaux provenant des gradiomètres (59) sont mémorisés en fonction du temps sous forme numérisée dans l'installation de traitement de données (67),
b) pendant l'intervalle de temps de mesure, les données concernant la position spatiale du dispositif de détection (7) par rapport à l'objet d'examen (1) sont déterminées au moyen de l'installation de traitement de données (67), sur la base des données délivrées par les moyens (88,89) servant à déterminer la position spatiale du dispositif de détection (7) par rapport au dispositif de support (5), et sont mémorisées en fonction du temps, sous forme numérisée, dans l'installation de traitement de données (67),
c) en partant d'un modèle de l'objet d'examen (1), sur la base des signaux délivrés par les gradiomètres (59) à des instants identiques pendant l'intervalle de temps de mesure et sur la base des données concernant la position spatiale du dispositif de détection (7) par rapport à l'objet d'examen (1), pour une source (Q) de signaux magnétiques, l'installation de traitement de données (67) calcule au choix,
i. la variation dans le temps de la densité de flux magnétique de la source (Q),
ii. la variation dans le temps de la densité de flux magnétique de la source (Q) dans une représentation correspondant à la disposition des gradiomètres,
iii. à un instant prédéterminé dans un intervalle de temps de mesure une carte des lignes de champ, qui contient les lignes de même densité du flux magnétique, qui sont associées à une source (Q) pour un plan pouvant être défini librement, ou
iv. à un instant prédéterminé pendant l'intervalle de temps de mesure, la position spatiale de la source (Q), et
d) les résultats sont délivrés par l'intermédiaire du dispositif de sortie (77).

20. Procédé suivant la revendication 19, caractérisé par le fait que pour le calcul devant être réalisé au moyen de l'installation de traitement de données (67), on part d'une sphère possédant une conductivité homogène en tant que modèle de l'objet d'examen (1).

21. Procédé suivant la revendication 19, caractérisé par le fait que pour le calcul qui doit être exécuté au moyen de l'installation de traitement de données (67), on prend pour base un demi-espace possédant une conductivité homogène en tant que modèle de l'objet d'examen (1).

22. Procédé suivant l'une des revendications 19 à 21, caractérisé par le fait qu'une succession de cartes de lignes de champ est délivrée à des instants successifs au cours de l'intervalle de temps de mesure.

23. Procédé suivant l'une des revendications 19 à 21, caractérisé par le fait qu'une image anatomique de l'objet d'examen (1), qui est formée au moyen d'un appareil de tomographie, est mémorisée dans l'installation de traitement de données (67), et que la délivrance des résultats s'effectue lorsqu'intervient la représentation de l'image anatomique de l'objet d'examen (1), sur laquelle la position de la source (Q) est portée à un instant prédéterminé pendant l'intervalle de temps de mesure.

24. Procédé suivant la revendication 23, caractérisé par le fait qu'une suite d'images anatomiques, sur lesquelles est marquée la position de la source (Q) est délivrée à des instants successifs pendant l'intervalle de temps de mesure.

25. Procédé suivant l'une des revendications 19 à 24, caractérisé par le fait qu'un être vivant est prévu en tant qu'objet d'examen (1).

26. Procédé suivant la revendication 25, caractérisé par le fait que les sens de l'être vivant sont stimulés pendant l'intervalle de temps de mesure.

27. Procédé suivant la revendication 26, caractérisé par le fait que les sens de l'être vivant sont stimulés optiquement.

28. Procédé suivant la revendication 26, caractérisé par le fait que les sens de l'être vivant sont stimulés acoustiquement.

29. Procédé suivant l'une des revendications 26 à 28, caractérisé par le fait que la délivrance de résultats s'effectue, au cours de l'intervalle de temps de mesure, à des instants auxquels les sens de l'être vivant sont stimulés.

30. Procédé suivant l'une des revendications 25 à 29, caractérisé par le fait que pendant l'intervalle de temps de mesure, une fonction physiologique périodique de l'être vivant est mesurée et est mémorisée, en fonction du temps, sous forme numérisée, dans l'installation de traitement de données (67).

31. Procédé suivant la revendication 30, caractérisé par le fait que la délivrance de résultats s'effectue à des instants auxquels la fonction physiologique de l'être vivant possède une valeur définie.
